(19)

Europäisches Patentamt

European Patent Office

Office européen des brevets

(11) **EP 3 699 923 A1**

(12) **EUROPÄISCHE PATENTANMELDUNG**

(43) Veröffentlichungstag:
26.08.2020 Patentblatt 2020/35

(21) Anmeldenummer: 19158317.8

(22) Anmeldetag: **20.02.2019**

(51) Int Cl.:
*G16H 40/40* (2018.01)          *G16H 40/60* (2018.01)
*G06F 21/00* (2013.01)          *H04L 29/08* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder:
• **Fresenius Medical Care Deutschland GmbH**
**61352 Bad Homburg (DE)**
• **Fresenius Medical Care AG & Co. KGaA**
**61352 Bad Homburg (DE)**

(72) Erfinder:
• **Hellhund, Jonas**
**60438 Frankfurt (DE)**
• **Lindemann, Robert**
**65193 Wiesbaden (DE)**
• **Peters, Arne**
**61352 Bad Homburg (DE)**
• **Fischer, Gerome**
**99947 Weberstedt (DE)**

(74) Vertreter: **Schwarz, Claudia et al**
**Schwarz + Kollegen**
**Patentanwälte**
**Hermann-Schmid-Straße 10**
**80336 München (DE)**

(54) **ABSICHERUNGSMECHANISMUS FUER DEN BETRIEB VON MEDIZINISCHEN GERAETEN UNTER VERWENDUNG VON VERBRAUCHSGEGENSTAENDEN IM UMFELD EINER DIALYSE**

(57) Die vorliegende Erfindung betrifft ein Absicherungsmodul (SM) und ein Dialysegerät (DG) oder eine Umkehrosmoseanlage (RO) mit einem solchen Absicherungsmodul (SM), ein Verfahren sowie ein System zur Absicherung eines Gerätes (DG, RO) im medizinischen Umfeld vor einem nicht autorisierten Betrieb des Gerätes, wobei die Autorisierung abhängig ist von einem Verknüpfungsereignis zwischen zwei Verknüpfungspartnern im Rahmen eines Gerätebetriebs, wobei alle oder ausgewählte Geräte (DG, RO) über ein Netzwerk datentechnisch verbunden sind und wobei einer der Verknüpfungspartner ein Gebrauchsgegenstand ist, der bei dem Gerätebetrieb verwendet wird. Dabei umfasst das System mehrere Geräte (DG, RO) eines Geräteverbundes, wobei jeweils ein Gerät (DG, RO) ein Absicherungsmodul enthält, umfassend:
- eine Einleseschnittstelle (I) zum Einlesen eines ersten Identifikators (ID-P) und eines zweiten Identifikators (ID-D);
- eine elektronische Verarbeitungseinheit (V), die ausgebildet ist, ein Absicherungsverfahren auszuführen;
- einen Speicher (MEM) zur Speicherung des berechneten Dokumentationswertes (W) in einer Distributed Ledger Struktur (DL);
- einer Schnittstelle zu dem Netzwerk, über das die Geräte in Datenaustausch stehen.

Fig. 1

EP 3 699 923 A1

**Beschreibung**

Die vorliegende Erfindung liegt auf dem Gebiet der Medizintechnik und betrifft den Betrieb von medizinischen Geräten, wie Dialysegeräten und Umkehrosmose-Anlagen, die mit Verbrauchsgegenständen (z.B. Einwegartikeln) betrieben werden. Die Erfindung betrifft insbesondere ein Verfahren zur Absicherung eines Gerätes gegen einen nicht-autorisierten Betrieb, ein Computerprogramm, ein Absicherungsmodul, ein medizinisches Gerät und ein System.

Der Betrieb von medizinischen Geräten weist insofern im Vergleich zu anderen nichtmedizinischen Betriebsverfahren eine Besonderheit auf, dass der Gerätebetrieb die Gesundheit eines Patienten betrifft. Insofern sind hier besondere Sicherungsmaßnahmen einzuhalten. Einige Betriebsverfahren sehen die Verwendung von Verbrauchsgegenständen (Disposables, wie z.B. einem Dialysator) vor. So erfolgt beispielsweise eine Dialysebehandlung bei einer Hämodialyse an einem Dialysegerät, das sich z.B. an einem Dialysezentrum befinden kann, üblicherweise derart, dass sich ein Patient an dem Dialysegerät registriert, um die Dialyse durchzuführen. Bei der Dialyse wird das Blut des Patienten durch einen extrakorporalen Kreislauf geführt, der z.B. als Einwegartikel ausgestaltet sein kann. Dazu kann ein vom Patienten mitgeführter oder für den Patienten bereitgestellter Satz von Verbrauchsgegenständen (z.B. Schlauchsatz mit Schlauchleitungen, Filtermodulen, Konnektoren, Tropfkammern etc.) verwendet werden.

Zur Behandlung selbst kann der Patient unterschiedliche Dialysegeräte verwenden, die sich in demselben oder auch in unterschiedlichen Dialysezentren (mitunter weltweit verteilt) befinden. Aus medizinischer Perspektive ist es wichtig, sicherzustellen, dass der Satz von Verbrauchsgegenständen auf vorgesehene Weise verwendet wird. Bestimmte Anwendungen erfordern es z.B., dass der Satz von Verbrauchsgegenständen nur einmal verwendet werden darf, während andere Anwendungen eine begrenzte Anzahl Wiederverwendungen zulassen. Auf jeden Fall ist jedoch sicherzustellen, dass der Satz von Verbrauchsgegenständen nur für genau einen Patienten verwendet wird (er darf somit nicht "ausgeliehen" werden an Mitpatienten). Eine Verwendung auf unterschiedlichen Maschinen kann jedoch erlaubt sein.

Stand der Technik

Bei bekannten Systemen aus dem Stand der Technik wurde die Autorisierung einer Verwendung von Verbrauchsgegenständen bisher nur unzureichend überprüft. In der Regel erfolgte dies durch das Behandlungspersonal manuell, z.B. durch manuelle Nachfrage bei dem Patienten. Es liegt auf der Hand, dass ein solches Verfahren mit teilweise gravierenden Nachteilen verbunden ist. Zum einen konnte eine Überprüfung vom Personal schlichtweg vergessen werden und somit unterbleiben, zum anderen sind die Informationsquellen nicht verlässlich. Dies beeinträchtigt die Sicherheit des Gerätebetriebs und kann sich nachteilig auf den gesamten Behandlungsprozess auswirken.

Aus der WO2015024647A2 ist es beispielsweise bekannt, den Gebrauchsgegenstand mit einem Sensor zu versehen, um Messwerte im Rahmen der Behandlung zu erfassen und verarbeiten zu können. Dabei kann es sich z.B. um einen Füllstandsensor eines Dialysatbehälters als Beispiel eines Gebrauchsgegenstandes oder um einen Temperatursensor zur Erfassung der Temperatur handeln.

Vergegenwärtigt man sich die praktische Situation bei einer Dialysebehandlung, so ist eine möglichst hohe Flexibilität für den Patienten gewünscht. Er soll mitunter die Möglichkeit haben, sich an einem beliebigen Dialysegerät eines beliebigen Dialysezentrums (z.B. weltweit verteilt) behandeln_zu lassen, um dort die Behandlung mit einem von ihm mitgeführten Satz von Verbrauchsgegenständen behandeln zu lassen. Die lokale Überprüfung der Verwendung der Verbrauchsgegenstände an einem spezifischen Gerät ist somit nicht ausreichend.

Zudem ist es wichtig, dass die Sicherheit der bezüglich des Patienten, der Verbrauchsgegenstände etc. gespeicherten Daten den Anforderungen an den Datenschutz und insbesondere dem Schutz von PHI-Daten (private or protected health information) genügt und die gespeicherten Daten nicht von unbefugten Personen einsehbar sind.

Der vorliegenden Erfindung liegt deshalb die Aufgabe zugrunde, eine technische Umsetzung anzugeben, mittels der die Sicherheit eines Gerätebetriebs erhöht und das Risiko für den Patienten gesenkt werden kann und dabei die Flexibilität für den Betreiber oder für den Patienten z.B. durch Auswahl eines beliebigen Behandlungsgerätes zu gewährleisten oder noch zu erhöhen. Ferner soll die Nachvollziehbarkeit eines Gerätebetriebs, insbesondere von ausgeführten Behandlungen, verbessert werden, um Rückschlüsse auf die jeweiligen Geräte zu ermöglichen und deren Dokumentation zu verbessern. Des Weiteren soll die technische Umsetzung effizient sein, so dass lange Wartezeiten aufgrund von Überprüfungsmaßnahmen am Gerät vermieden werden können.

Diese Aufgabe wird durch die Gegenstände der unabhängigen Ansprüche gelöst, insbesondere durch ein Verfahren, ein Computerprogramm, ein Absicherungsmodul, ein medizinisches Gerät mit einem solchen Absicherungsmodul und durch ein System. Vorteilhafte Ausführungsformen der Erfindung sind in den abhängigen Ansprüchen und der nachfolgenden Beschreibung beschrieben.

Gemäß einem ersten Aspekt bezieht sich die Erfindung somit auf ein Verfahren zur Absicherung eines Gerätes im medizinischen Umfeld vor einem nicht autorisierten Betrieb des Gerätes, wobei die Autorisierung abhängig ist von einem Verknüpfungsereignis zwischen zwei oder mehr Verknüpfungspartnern im Rahmen eines Gerätebetriebs, wobei das Gerät zumindest zeitweise zum Datenaustausch mit andern Geräten an ein Netzwerk angeschlossen ist, und wobei einer der Verknüpfungspartner ein Gebrauchsgegenstand ist, der bei dem Gerätebetrieb verwendet wird, mit folgenden Verfahrensschritten:

- Einlesen eines ersten (digitalen) Identifikators, der einen ersten Verknüpfungspartner eineindeutig identifiziert und Einlesen eines zweiten (digitalen) Identifikators, der einen zweiten Verknüpfungspartner eineindeutig identifiziert. Das Einlesen kann über eine Einleseschnittstelle des Gerätes ausgeführt werden;
- Anwenden zumindest einer ersten Rechenvorschrift auf den eingelesenen ersten Identifikator und Anwenden einer zweiten Rechenvorschrift auf den zweiten Identifikator zur Berechnung eines Dokumentationswertes, der ein Verknüpfungsereignis zwischen dem ersten und zweiten Verknüpfungspartner eindeutig repräsentiert; zur Berechnung des Dokumentationswertes kann eine dritte Rechenvorschrift angewendet werden; die Berechnung erfolgt auf einer digitalen Verarbeitungseinheit, die Bestandteil eines Absicherungsmoduls sein kann;
- Synchronisiertes Speichern des berechneten (und optional: validierten) Dokumentationswertes in Speichern einer verteilten Speicherstruktur von allen oder ausgewählten Geräten des Netzwerkes (vorzugsweise von einer Mehrzahl von Geräten).

Vorzugsweise ist es sichergestellt, dass allen teilnehmenden Geräten die Rechenvorschriften bekannt sind. Deshalb ist in einer bevorzugten Ausführungsform der Erfindung eine Vorbereitungsphase vorgesehen, die dazu dient, die verwendeten Rechenvorschriften, umfassend die erste, zweite und ggf. dritte auszutauschen, um die Geräte in die Lage zu versetzen, Daten in die verteilte Datenstruktur schreiben und auslesen zu können.

Gemäß einem weiteren Aspekt bezieht sich die Erfindung auf ein Verfahren zur fälschungssicheren Dokumentation eines Gerätebetriebs eines medizintechnischen Gerätes, das mit einem Gebrauchsgegenstand (für einen bestimmten Patienten) betrieben werden soll. Dazu wird ein Verknüpfungsereignis in digitaler Form in einen Dokumentationswert repräsentiert. Der Dokumentationswert repräsentiert auf fälschungssichere Weise die Verwendung eines bestimmten Gebrauchsgegenstandes (z.B. Dialysator) für einen bestimmten Patienten an einem bestimmten Gerät (insbesondere Dialysegerät) oder die Verwendung eines bestimmten Gebrauchsgegenstandes (z.B. Membran) an einem bestimmten Gerät (insbesondere Umkehrosmoseanlage). Die fälschungssichere Dokumentation erfolgt durch das Speichern des berechneten Dokumentationswertes. Das Verfahren weist ebenso die oben beschriebenen Verfahrensschritte des Einlesens, des Anwendens und des synchronisierten Speicherns auf.

Die im Folgenden geschilderten alternativen Ausführungsform der Erfindung können sich auf beide Varianten des Verfahrens beziehen.

In einer vorteilhaften Weiterbildung kann das Verfahren ein Validieren des berechneten Dokumentationswertes seitens einer Gruppe von Geräten in dem Netzwerk durch Anwendung eines Konsensus-Algorithmus umfassen, so dass nur bei erfolgreicher Validierung das Speichern und/oder ein Autorisieren des Gerätebetriebs ausgeführt werden.

In einer vorteilhaften Weiterbildung des Verfahrens kann das Verfahren bei erfolgreicher Validierung ein Autorisieren des Gerätebetriebs für den bzw. mit dem ersten und zweiten Verknüpfungspartner umfassen bzw. das Gerät freischalten. Andernfalls, bei fehlender Validierung, kann das Gerät zumindest für die Verwendung mit den jeweiligen Verknüpfungspartnern gesperrt werden.

Das Absicherungsverfahren ist effektiv, kann auf verteilten Smart Devices (z.B. IoT Devices, Iot: Internet of Things) ohne Notwendigkeit einer zentralen Instanz ausgeführt werden und weist zudem eine hohe Manipulationssicherheit auf.

Das Validieren des berechneten Dokumentationswertes kann auf einer konfigurierbaren Gruppe von Geräten ausgeführt werden. Die Gruppe wird bevorzugt aus einer Mehrzahl der Geräte gebildet. Es kann konfiguriert sein, dass grundsätzlich alle Geräte für die Validierung verwendet werden oder nur ausgewählte (z.B. mit ausreichender Rechenleistung).

Die Verknüpfungspartner sind mit physikalischen Kennzeichnungen ausgebildet bzw. mit Labels gekennzeichnet, über die sie eineindeutig identifiziert werden können. Die Kennzeichnungen können z.B. als Code (z.B. als Barcode oder QR-Code) ausgebildet sein. Diese Kennzeichnung ist auf eineindeutige Weise einem Identifikator zugeordnet oder kann in diesen mittels einer Abbildungsvorschrift transformiert werden. Der Identifikator muss einem Gerät in dem Netzwerk auf geeignete Weise zur Verfügung gestellt und übermittelt werden, damit das Absicherungsverfahren ausführbar ist.

In einer Ausführungsform der Erfindung kann der Verknüpfungspartner einer elektronischen Vorrichtung (Handy/Smartphone des Patienten) zugeordnet sein, in der die jeweilige digitale Repräsentation oder Instanz der den Verknüpfungspartner identifizierenden Kennzeichnung hinterlegt ist (z.B. Patienten-ID in einer App). Diese digitale Repräsentation kann dann als Patienten-Identifikator dem Gerät zur weiteren Verarbeitung übermittelt werden.

In einer weiteren Ausführungsform der Erfindung kann zum Beispiel ein Gebrauchsgegenstand mit einer physikalischen Kennzeichnung (z.B. Barcode oder RFID-Chip) versehen sein. Diese Kennzeichnung kann über ein entsprechendes Lesemittel (Scanner, z.B. Barcodescanner oder RFID-Reader) eingelesen und in eine digitale Instanz transformiert werden, die fortan als digitaler Identifikator für den Gebrauchsgegenstand fungiert. Das Lesemittel kann in das Gerät (z.B. Dialysegerät) integriert oder diesem zugeordnet sein. Es ist auch möglich, einen den jeweiligen Verknüpfungspartner identifizierenden Identifikator direkt und manuell (z.B. über eine Benutzerschnittstelle) an dem Gerät einzugeben.

In einer ersten Ausführungsform der Erfindung ist das Gerät (z.B. Dialysegerät oder Umkehrosmoseanlage) selbst mit einem Absicherungsmodul zur Ausführung des Verfahrens ausgebildet und verfügt über Schnittstellen zum Einlesen des ersten und zweiten Identifikators.

In einer zweiten Ausführungsform der Erfindung ist das Gerät nicht selbst mit einem Absicherungsmodul ausgebildet, sondern steht mit diesem zumindest zeitweise oder phasenweise in Datenaustausch. Das Absicherungsmodul ist dann auf einem anderen Gerät ausgebildet, das als Gateway oder Intermediär fungiert. Der Begriff "Gateway" ist hier im Sinne von intermediärem Knoten zu verstehen und kann z.B. als Smartphone oder Tablet oder als mobiles Endgerät eines anderen Typs ausgebildet sein.

Die Verknüpfungspartner senden 'ihren' Identifikator über ein vorzugsweise kabelloses Netzwerk (z.B. Funk, WLAN, optisch etc.) an ein Gerät des Netzwerkes. Der Verknüpfungspartner (z.B. das Disposable) kann seine physikalische Kennzeichnung z.B. als Barcode an ein Lesemittel bzw. Einlesevorrichtung (z.B. Barcodescanner) senden, die den eingelesenen Code in einen digitalen Identifikator überführt und an das Dialysegerät sendet.

In dieser Ausführungsform der Erfindung können somit zwei unterschiedliche Netzwerke implementiert sein: Ein (erstes) Netzwerk zwischen den Verknüpfungspartnern (z.B. Disposable mit RFID Chip) und deren zugeordneten Vorrichtungen bzw. Lesemitteln (z.B. RFID Scanner) und ein anderes (zweites) Netzwerk, das zum Datenaustausch zwischen den Geräten dient, auf denen das Absicherungsverfahren angewendet werden soll (Dialysegerät oder Gateway) und deren Speicher eine verteilte Datenstruktur bilden. Letzteres kann insbesondere auf einem IP-Protokoll basieren.

Im Folgenden werden die Begrifflichkeiten der Anmeldung und vorteilhafte Ausführungsformen der Erfindung spezifiziert.

Ein Gerät kann ein medizinisches Gerät sein (z.B. ein Dialysegerät) oder ein nichtmedizinisches, das im medizinischen Umfeld verwendet wird (z.B. eine Umkehrosmose-Anlage, die im Folgenden aufgrund des englischen Begriffs "reverse osmosis" kurz als RO-Anlage bezeichnet wird) oder ein Gerät, das im Rahmen einer therapeutischen Apherese mit einem Gebrauchsgegenstand betrieben wird. Bei dem medizinischen Gerät kann es sich z.B. um ein Hämodialysegerät, ein Hämodiafiltrationsgerät oder ein Peritonealdialysegerät handeln. Das Dialysegerät wird vorzugsweise in einem Verbund von Geräten betrieben. So kann das Dialysegerät mit anderen parallel in einem Dialysezentrum betrieben werden. Das Dialysegerät kann aber auch als Heimdialysegerät betrieben werden. Dabei werden weltweit unterschiedliche Dialysezentren betrieben. Ein wesentlicher Vorteil der vorgeschlagenen Lösung ist darin zu sehen, dass die Geräte von unterschiedlichen Herstellern stammen und/oder von unterschiedlichen Betreibern betrieben werden können, um an dem Absicherungsverfahren teilnehmen zu können.

Das Gerät kann auch ein Heim-Hämodialysegerät sein. Gerade dort ist eine Wiederverwendung eines Dialysators aus wirtschaftlichen Gründen geradezu notwendig. Auf der anderen Seite ist der Patient aber weitgehend ohne Aufsicht von medizinischem Fachpersonal. Dort könnte also leichter eine Überschreitung der gesundheitlich zulässigen Wiederholungszahl unbemerkt auftreten (oder aus Kostengründen sogar beabsichtigt), so dass die erfindungsgemäße Absicherung bzw. Überprüfung dahingehend, dass nicht zu viele Wiederholungen auftreten, äußerst hilfreich ist. Zugleich erweist es sich für Krankenkassen und Herstellerunternehmen als wünschenswert, dass sichergestellt werden kann, dass es fälschungssicher dokumentiert wird, wenn eine unzulässige Wiederholungszahl auftritt. So können ggf. Haftungsfragen geklärt und das Einhalten von Behandlungsplänen bezeugt werden.

Ein Gebrauchsgegenstand kann ein Verbrauchsgegenstand sein. Der Gebrauchsgegenstand kann als Einweg- oder Mehrwegartikel für ein Dialysegerät ausgebildet sein, wie z.B. einen Dialysator, eine Blutleitung oder eine Dialysatleitung, die als Schlauchleitungen ausgebildet sein können. Der Gebrauchsgegenstand kann ebenso eine Membran oder einen anderen Gebrauchsgegenstand für eine Umkehrosmoseanlage betreffen. In einem weiteren Anwendungsbeispiel kann der Gebrauchsgegenstand auch ein Dialysefilter wie ein Dialysator für Hämodialyse und/oder Hämodiafiltration sein. In einem weiteren Anwendungsbeispiel kann der Gebrauchsgegenstand auch ein Adsorber für eine therapeutische Apherese sein. Der Gebrauchsgegenstand kann in vorteilhaften Weiterbildungen der Erfindung auch ein sonstiger Gegenstand oder ein weiteres Gerät sein, das mit dem Gerät verwendet oder betrieben wird.

Die Autorisierung basiert auf einer automatischen Überprüfung der Zulässigkeit einer Verwendung eines Gebrauchsgegenstandes für das medizintechnische Gerät und/oder für einen Patienten. Dabei können je nach Anwendung unterschiedliche Zulässigkeitsvoraussetzungen konfiguriert werden, wie z.B. nur einmalige Verwendung oder mehrfache Verwendung des Gebrauchsgegenstandes mit einer konfigurierbaren Begrenzung der Anzahl von zulässigen Verwendungen oder anderen Zulässigkeitskriterien (z.B. maximale Standzeit, maximale Betriebsdauer etc.) für den jeweiligen Verknüpfungspartner (z.B. Dialysemaschine). Die Autorisierung kann eine Freischaltung des Gerätes und/oder eine Sperrung für den geplanten Betrieb des Gerätes mit dem Gebrauchsgegenstand umfassen. Die Autorisierung kann noch weitere Zulässigkeitsprüfungen umfassen (z.B. siehe oben: Die Überprüfung von Zulässigkeitsvoraussetzungen).

Ein erster Verknüpfungspartner kann ein Patient sein, der an einem Dialysegerät behandelt wird und ein zweiter Verknüpfungspartner kann ein Gebrauchsgegenstand (Dialysator, Schlauchset für den extrakorporalen Blutkreislauf etc.) zur Verwendung mit dem Dialysegerät sein. Alternativ kann der erste Verknüpfungspartner eine Umkehrosmoseanlage und der zweite Verknüpfungspartner eine Membran oder ein anderer Gebrauchsgegenstand für die Umkehrosmoseanlage sein. Ein Verknüpfungspartner kann eine ihn eineindeutig identifizierende Kennzeichnung (z.B. in Form eines QR-Codes) aufweisen. Diese Kennzeichnung ist auf eineindeutige Weise einem digitalen Identifikator zugeordnet. So hat z.B. jeder Patient einen ihn identifizierenden Patienten-Identifikator und jeder Gebrauchsgegenstand einen ihn identifizierenden Disposable-Identifikator.

Die Geräte können Kommunikationspartner in einem Netzwerk sein, die über ein vorzugsweise drahtloses Netzwerk (z.B. WLAN, nach einem Protokoll gemäß dem Standard der IEEE-802.11-Familie) in Datenaustausch stehen und insbesondere Daten in eine verteilte Speicherstruktur schreiben und lesen können.

Der erste und zweite Identifikator dient jeweils zur eindeutigen Identifikation des ersten bzw. zweiten Verknüpfungspartners. Bei dem Identifikator handelt es sich vorzugsweise um einen digitalen bzw. elektronisch verarbeitbaren Identifizierungscode, der einer direkt auf dem Verknüpfungspartner (Gebrauchsgegenstand ist gekennzeichnet) aufgebrachten oder diesem zugeordneten (Patient hat eine Patientenkarte, die ihn identifiziert) Kennzeichnungen bijektiv zugeordnet ist. Der Identifikator kann z.B. über einen Zufallsgenerator erzeugt und mit einem Algorithmus derart bearbeitet sein, dass sichergestellt ist, dass eine eineindeutige Zuordnung zwischen Identifikator und Verknüpfungspartner gewährleistet ist, so dass genau ein Identifikator genau einem Verknüpfungspartner zugeordnet ist.

Die Kennzeichnung ist ein physikalisches Label, Tag oder Token und kann direkt auf dem Gebrauchsgegenstand aufgebracht sein, z.B. als aufgeklebtes elektronisches Label. Eine Kennzeichnung kann beispielsweise Zahlen, Buchstaben, Sonderzeichen sowie Kombinationen aus diesen umfassen. Die Kennzeichnung kann auch einem Verknüpfungspartner eindeutig zugeordnet sein. Die Kennzeichnung wird über entsprechende Schnittstellen bzw. Einlesemittel erfasst bzw. eingelesen (Datenschnittstelle oder optischer Sensor, Scanner). Die Kennzeichnung kann eine Kennung sein, die zur eindeutigen Identifizierung des Verknüpfungspartners dient (Barcode, QR-Code) und ist elektronisch erfassbar und auswertbar. Wenn die Kennzeichnung eingelesen ist, dann kann sie mittels einer eineindeutigen Abbildungsvorschrift einem Identifikator zugeordnet sein.

Ein Verknüpfungsereignis repräsentiert die Verknüpfung von zumindest zwei Verknüpfungspartnern. Mit "Verknüpfung" ist hier ein gemeinsamer Betrieb oder die Verwendung eines Gebrauchsgegenstands durch einen Verbraucher bei bzw. während einem/eines Betrieb(s) gemeint. Das Gerät kann bei dem Verknüpfungsereignis mitwirken. Das Verknüpfungsereignis kann während einer Aufrüstung, Vorbereitung, Inbetriebnahme und/oder während des Betriebs des Gerätes erfolgen. Mit anderen Worten repräsentiert die Verknüpfung z.B. die Verwendung eines bestimmten Dialysators oder Schlauchsets als Gebrauchsgegenstand in dem Dialysegerät für den bestimmten Patienten oder die Verwendung der bestimmten Membran in der Umkehrosmoseanlage. Zunächst wird ein Verknüpfungsereignis intendiert oder angefragt von zumindest einem der Verknüpfungspartner. Dies kann z.B. ausgelöst werden, indem der Patient seine ID-Karte in eine Aufnahmevorrichtung an dem Gerät einführt und/oder entsprechende Benutzereingaben auf einer Benutzeroberfläche eingibt und/oder indem ein Identifikator des dabei zu verwendenden Gebrauchsgegenstandes eingelesen wird. Um die Sicherheit zu erhöhen, wird das Verknüpfungsereignis zumindest fälschungssicher abrufbar dokumentiert und gespeichert. Des Weiteren kann es vorgesehen sein, dass die Verknüpfung nicht gleich umgesetzt wird, sondern dass zunächst eine Überprüfung erfolgt, ob die geplante Verwendung auch autorisiert ist. Dazu wird auf die verteilte Speicherstruktur mit einem definierten Zugriffsalgorithmus zugegriffen. Ein Verknüpfungsereignis kann auf Basis von konfigurierbaren Kriterien erfasst werden. Eine Erfassung eines Verknüpfungsereignisses kann zeitbasiert (z.B. zeitlich getaktet erfolgen oder nach einer bestimmten Anzahl von Verwendungen) oder ereignisbasiert (z.B. nach dem Einlegen oder dem Ausführen einer bestimmten Handlung/Aktion). Das Erfassen eines Verknüpfungsereignisses kann das Validieren umfassen. Es kann aber auch sein, dass das Erfassen das Einlesen, Anwenden und Validieren umfasst. Mit anderen Worten werden diese Verfahrensschritte nur ausgeführt, wenn ein Trigger Kriterium erfüllt ist (zeitbasiert, ereignisbasiert). In einer Ausführungsform der Erfindung können die Identifikatoren eingelesen und die Rechenvorschriften angewendet werden.

Bei der ersten, zweiten, dritten und/oder weiteren Rechenvorschrift und/oder der Verknüpfungsfunktion kann es sich um eine auf einer digitalen Recheneinheit auszuführende Vorschrift handeln. In einer bevorzugten Ausführungsform der Erfindung sind diese Rechenvorschriften identisch. In einer einfachen Ausführung werden die beiden Identifikatoren mit einander verhasht und dadurch wird der Wert erzeugt. In diesem einfachen Fall erfolgt keine weitere Operation. Hier wird also ein Identifikator als Seed für einen Hash des jeweils anderen Identifikators verwendet.

Die Rechenvorschriften, insbesondere die erste und die zweite Rechenvorschrift können aber auch unterschiedlich ausgebildet sein. Die erste Rechenvorschrift wird auf den ersten Identifikator angewendet, um ein erstes Teilergebnis zu berechnen und die zweite Rechenvorschrift wird auf den zweiten Identifikator angewendet, um das zweite Teilergebnis zu berechnen. Die Verknüpfungsfunktion dient zur Berechnung des Dokumentationswertes aus dem ersten und zweiten Teilergebnis. Die Rechenvorschrift und/oder die Verknüpfungsfunktion können insbesondere kryptologisch sein. Die Rechenvorschrift und/oder die Verknüpfungsfunktion können z.B. eine kryptologische Hashfunktion oder eine einfache Hashfunktion sein, wie z.B. die Anwendung eines SHA, MD5 oder anderen Algorithmus.

Der Dokumentationswert wird aus dem ersten Teilergebnis (Anwendung der ersten Rechenvorschrift auf den ersten Identifikator) und aus dem zweiten Teilergebnis (Anwendung der zweiten Rechenvorschrift auf den zweiten Identifikator) berechnet. Auf das erste und das zweite Teilergebnis kann die dritte Rechenvorschrift angewendet werden, um den Dokumentationswert zu errechnen. In alternativen Ausführungsformen der Erfindung kann der Dokumentationswert darüber hinaus noch weitere Daten, insbesondere Messdaten, die während der Geräteverwendung mit den Verknüpfungspartnern (z.B. mit dem jeweiligen Disposable während einer Dialysebehandlung) anfallen, umfassen. Weitere Daten, die mit dem erfindungsgemäßen Verfahren sicher dokumentiert werden, können sein:

- Geräte ID, Komponenten IDs, Standortdaten, Behandlungsdaten wie mittlerer Blutdruck, Clearance, mittlere Leitfähigkeit des Dialysats (Na Konzentration), etc.
- Information zur Kommunikationsanbindung (Latenz, IP Adresse, Mac Adresse, physikalische Location). Das Tracking dieser Daten hat den technischen Vorteil, dass die Kommunikationsverbindung in ihrer Güte vorhersagbar und/oder das System damit insgesamt zuverlässiger ausgebildet werden kann.
- Ausführungsdauer des erfindungsgemäßen Verfahrens auf diesem Gerät. Dabei kann die Ausführungsdauer für verschiedene Hardware unterschiedlich sein). Der Vorteil ist in der verbesserten Vorhersagbarkeit begründet.
- Die vorstehend genannte Liste von weiteren Daten ist erweiterbar. Im Grunde sind alle Behandlungs- sowie Umgebungsdaten denkbar, wie z.B. Blutdruck sowie Medikamentengabe wie Heparin, Epo etc. Zudem können Alarme Teil der weiteren Daten sein.

Bei dem Netzwerk handelt es sich um ein Netzwerk zum Austausch von digitalen Daten zur Ausführung des Absicherungsverfahrens. Das Netzwerk kann vorzugsweise als Peer to Peer Netzwerk ausgebildet sein und bietet den Kommunikationsteilnehmern die Möglichkeit, Dienste und Ressourcen von den anderen Kommunikationsteilnehmern auf gleichberechtigte Weise in Anspruch zu nehmen. Das Netzwerk ist offen für alle Netzwerkteilnehmer. Dies stellt kein Sicherheitsrisiko dar, da die in der verteilten Speicherstruktur abgelegten Daten gesichert abgelegt und vorzugsweise kryptologisch verschlüsselt sind und somit selbst bei einem Abhören nicht im Klartext vorliegen.

Unter einem "Programm" bzw. "Programminstruktionen" wird hier jede Art von Computerprogramm verstanden, das maschinenlesbare Instruktionen zur Steuerung einer Funktionalität des Computers umfasst. Das Computerprogramm kann auf einem Datenträger als ausführbare Programmdatei, häufig im sogenannten Maschinencode gespeichert sein, die zur Ausführung in den Arbeitsspeicher des Rechners geladen wird. Das Programm wird als Abfolge von Maschinen-, d. h. Prozessorbefehlen von dem oder den Prozessoren des Computers verarbeitet und damit ausgeführt. Das Programm kann als ausführbarer Code, als Quellcode oder als interpretierter Code vorliegen.

Unter einer "Schnittstelle" wird eine Schnittstelle verstanden, über die Daten empfangen und gesendet werden können (Kommunikationsschnittstelle). Die Kommunikationsschnittstelle kann kontaktbehaftet oder kontaktlos konfiguriert sein. Bei der Kommunikationsschnittstelle kann es sich um eine interne Schnittstelle oder um eine externe Schnittstelle handeln, die beispielsweise mittels eines Kabels oder kabellos mit einem zugeordneten Gerät verbunden ist. Die Kommunikation kann über ein Netzwerk erfolgen. Unter einem "Netzwerk" wird hier jedes Übertragungsmedium mit einer Anbindung zur Kommunikation verstanden, insbesondere eine lokale Verbindung oder ein lokales Netzwerk, insbesondere ein Local Area Network (LAN), ein privates Netzwerk, insbesondere ein Intranet, und ein virtuelles privates Netzwerk (Virtual Private Network - VPN). Beispielsweise kann ein Computersystem eine Standardfunkschnittstelle zur Anbindung an ein WLAN aufweisen. Ferner kann es sich um ein öffentliches Netzwerk, wie beispielsweise das Internet handeln. Je nach Ausführungsform kann die Kommunikation auch über ein Mobilfunknetz erfolgen.

Unter einem "Speicher" werden hier sowohl flüchtige als auch nichtflüchtige elektronische Speicher bzw. digitale Speichermedien verstanden. Unter einem "nichtflüchtigen Speicher" wird ein elektronischer Speicher zur dauerhaften Speicherung von Daten verstanden. Ein nichtflüchtiger Speicher kann als nichtänderbarer Speicher konfiguriert sein, der auch als Read-Only Memory (ROM) bezeichnet wird, oder als änderbarer Speicher, der auch als Non-Volatile Memory (NVM) bezeichnet wird. Insbesondere kann es sich hierbei um ein EEPROM, beispielsweise ein Flash-EEPROM, kurz als Flash bezeichnet, handeln. Ein nichtflüchtiger Speicher zeichnet sich dadurch aus, dass die darauf gespeicherten Daten auch nach Abschalten der Energieversorgung erhalten bleiben. Unter einem "flüchtigen elektronischen Speicher" wird hier ein Speicher zur vorübergehenden Speicherung von Daten verstanden, welcher dadurch gekennzeichnet ist, dass alle Daten nach dem Abschalten der Energieversorgung verloren gehen. Insbesondere kann es sich hierbei um einen flüchtigen Direktzugriffsspeicher, der auch als Random-Access Memory (RAM) bezeichnet wird, oder einen flüchtigen Arbeitsspeicher des Prozessors handeln.

Unter einer "Verarbeitungseinheit" wird ein Elektronikmodul verstanden. Es handelt sich typischerweise um eine digitale Verarbeitungseinheit, die als Softwaremodul implementiert sein kann, z.B. als Teil von virtuellen Maschinen. Die Verarbeitungseinheit kann z.B. als Prozessor zur computerbasierten, automatischen Ausführung von Befehlen ausgebildet sein und eine Logikschaltung zur Ausführung von Programminstruktionen umfassen. Die Logikschaltung kann auf einem oder mehreren diskreten Bauelementen implementiert sein, insbesondere auf einem Chip. Insbesondere wird unter einem "Prozessor" ein Mikroprozessor oder ein Mikroprozessorsystem aus mehreren Prozessorkernen und/oder mehreren Mikroprozessoren verstanden.

Das vorstehend in Bezug auf die Verarbeitungseinheit Gesagte, gilt ebenso entsprechend auch für das Absicherungsmodul. Auch dieses kann als Softwaremodul, z.B. als Teil einer virtuellen Maschine, ausgebildet sein.

In einer bevorzugten Ausführungsform der Erfindung kann der Dokumentationswert zusätzlich weitere Informationen zu dem jeweiligen Verknüpfungsereignis bereitstellen, die z.B. eine Aussage darüber zulassen, ob die Aufrüstung der Dialysemaschine ausreichend und angepasst ist, mit den jeweiligen Teilen des extrakorporalen Kreislaufs in Eingriff gebracht werden zu können. Darüber hinaus kann der Dokumentationswert noch weitere Metadaten zu dem Verknüpfungsereignis bereitstellen (Zeitpunkt, Dauer, Anzahl von bisherigen Verwendungen, ggf. Wiederholungsrate, Anzahl verbleibender Verwendungen etc.).

Ein Konsensus-Algorithmus wird verteilt auf mehreren Knoten bzw. Geräten ausgeführt und dient zur Herstellung eines Konsenses bzw. einer Übereinstimmung in Bezug auf einen Wert und damit zum Validieren des Wertes. Er wird auf allen oder ausgewählten Geräten oder Teilnehmern des Dokumentationsverfahrens einheitlich ausgeführt, um eine übereinstimmende Beurteilungsgrundlage zu schaffen. Der Konsensus-Algorithmus wird nach nach einem Konsens-Protokoll betrieben und kann als Proof-of-Work- oder (delegated) Proof-of-Stake-Algorithmus ausgebildet sein. Ein Konsensus-Algorithmus bezieht sich grundsätzlich auf einen Mechanismus zum Erreichen einer einheitlichen Vereinbarung (Konsens) über den Zustand (Status) des dezentralen Netzes. Ein Konsens-Algorithmus erleichtert die Überprüfung und Validierung von Informationen, die in die verteilte Datenstruktur (DL, Hauptbuch) geschrieben (gespeichert) werden. Dadurch wird sichergestellt, dass nur authentische Daten in der verteilten Datenstruktur aufgezeichnet werden. Die Regeln zur Anwendung in dem Konsens-Protokoll sind hart codiert, einvernehmlich unter allen Teilnehmern vereinbart und stellen die einzige Quelle von Überprüfungsregeln dar, so dass sichergestellt werden kann, dass die gespeicherten Informationen vertrauenswürdig sind, ohne dass eine zentrale Instanz erforderlich ist. Für weitere Details sei auf das folgende Paper verwiesen "Blockchain consensus protocols in the wild", C Cachin, M Vukolic - arXiv preprint arXiv:1707.01873, 2017 - arxiv.org (v2). Erst wenn ein Dokumentationswert validiert werden konnte, kann er auf synchronisierte Weise in allen Speichern der Verknüpfungspartner, die eine verteilte Speicherstruktur bilden, gespeichert werden. Der Konsensus-Algorithmus umfasst einen Zugriff auf die verteilte Speicherstruktur. Das Validieren mittels des Konsensus-Algorithmus' dient zur lokalen Suche, ob der jeweilige Wert schon einmal und wenn ja, wie oft, abgespeichert worden ist. In einer Erweiterung können dabei noch weitere Schritte ausgeführt werden, wie z.B. die Anwendung von in einer Regelbasis vorgehaltenen Regeln und / oder darauf basierender computer-implementierter Algorithmen. Das Validieren erfolgt ähnlich wie bei der klassischen Blockchain, indem ein Teilnehmer im Netz (Gateway oder Gerät) versucht, einen berechneten Wert (entsprechend bspw. einer Transaktion bei Bitcoin) zu verifizieren, indem versucht wird, dieselbe (identische) Signatur zu erzeugen. Die Suche in der Distributed Ledger kann direkt nach der Signatur erfolgen und ist damit schneller im Vergleich zu einer Suche über Speicheradressen und Blocknamen etc.

Es kann konfiguriert sein, dass ein Gerätebetrieb nur dann aktivierbar ist, wenn er mit den jeweiligen Verknüpfungspartnern als autorisiert evaluiert worden ist. Für die Evaluation ist eine erfolgreiche Validierung (wie vorstehend erläutert auf Basis des berechneten Dokumentationswertes) eine zwingende Voraussetzung. Für die Autorisierung des Gerätes können unter Umständen noch weitere Prüfungen durchgeführt werden (zeitbasiert, ereignisbasiert etc.). Damit kann der Gerätebetrieb auf flexible und verteilte Weise gegen einen unautorisierten Betrieb abgesichert werden.

Das synchronisierte Speichern bedeutet, dass ein Dokumentationswert, sofern er erfolgreich validiert werden konnte, in allen Speicher der Geräte des Absicherungsverfahrens bzw. des Absicherungssystems identisch gespeichert wird. Die Geräte können z.B. eine Gruppe von Dialysegeräten und/oder Umkehrosmoseanlage sein. Die Geräte des Validierungsverfahrens müssen nicht notwendigerweise mit den Verknüpfungspartnern übereinstimmen. Damit kann der Kernschritt zur Absicherung des Gerätebetriebs, nämlich die Validierung, nicht direkt auf den Verknüpfungspartnern, sondern auf anderen computer-basierten Knoten bzw. Instanzen ausgeführt werden, z.B. auf Gateways oder den Geräten zugeordneten Knoten. Alle Speicher der Geräte sind immer auf demselben Stand und verfügen über denselben Speicherstatus. Damit entsteht eine verteilte Speicherstruktur.

Bei der verteilten Speicherstruktur handelt es sich vorzugsweise um eine distributed ledger Struktur (DLT-Struktur), die auf allen potentiellen Geräten und Teilenehmern des Absicherungsverfahrens implementiert sein kann. Die Geräte sind dazu mit Mitteln (darunter Speichermittel und Sofwaremittel) ausgestattet, um an der distributed ledger Struktur teilzunehmen. Ein distributed ledger System (verteiltes Hauptbuchsystem) kann Daten speichern, die auf verschiedene Transaktionen bzw. Berechnungen zwischen bzw. von mehreren Rechengeräten (z.B. mehreren Dialysegeräten) hinweisen. Das verteilte Ledgersystem kann als Blockchain-Ledgersystem ausgeführt werden, das eine Vielzahl von "Blöcken" umfasst, die jeweils eine oder mehrere diskrete Transaktion(en) darstellen, die zwischen den Rechengeräten stattfinden. Jeder Block kann Daten umfassen, die mit einem zuvor erzeugten Block verknüpft sein können, wodurch eine vollständige Kette zwischen der Erzeugung von Daten, die im verteilten Ledger gespeichert sind, und der späteren Verwendung derselben Daten entsteht (z.B. zum Aufbau einer vollständigen Kette von Verwendungen von Verbrauchsgegenständen für einen Patienten in einem Gerät). Die in den verschiedenen Transaktionen/Blöcken gespeicherten Daten können verschlüsselt, mit einem Passwort versehen oder anderweitig vor unbefugtem Zugriff geschützt sein (z.B. Lesezugriff, Schreibzugriff und/oder Löschzugriff). In einem nicht einschränkenden Beispiel können Informationen, die in den verschiedenen Blöcken gespeichert sind, irreversibel gehasht werden, so dass die gehashten Daten verwendet werden können, um die Echtheit von Transaktionen zu überprüfen, wobei sichergestellt ist, dass die gehashten Daten nicht rückentwickelt werden können, um substantielle Informationen allein basierend auf den gehashten Informationen zu ermitteln. Damit können sicherheitskritische medizinische Daten auf sichere Weise verteilt werden. Darüber hinaus können Daten, die zwischen verschiedenen Computern übertragen werden, verschlüsselt werden (z.B. durch Verwendung von öffentlichen/privaten Schlüsselpaaren zum digitalen Signieren und/oder Verifizieren von Daten), so dass innerhalb der Blockkette gespeicherte Blöcke von mehreren Geräten verifiziert werden können, die Zugriff auf die öffentlichen und/oder privaten Schlüssel haben. Nach Überprüfung (Validierung) der in der Blockchain zu speichernden Daten können die Daten wie oben beschrieben gehasht und gespeichert werden. Für weitere Details zu den Begrifflichkeiten verteiltes System / distributed ledger sei auf A. S. Tanenbaum, M. van Steen: Verteilte Systeme, 2007, insbesondere Kapitel 7.5 verwiesen. Auf den einzelnen verteilten Recheninstanzen bzw. Computern laufen Prozesse zur Ausführung des erfindungsgemäßen Dokumentationsverfahrens. Prozesse auf den unterschiedlichen Recheninstanzen in dem verteilten System kommunizieren miteinander durch das Senden von Nachrichten. Dabei müssen die Prozesse Regeln einhalten, z. B. bezüglich der Formate, der Bedeutungen der ausgetauschten Nachrichten und der erforderlichen Aktionen zur Übermittlung. Diese Regeln werden als Protokoll bezeichnet.

Mit dem erfindungsgemäßen Absicherungsverfahren werden Blöcke (Datenblöcke) in einer Vielzahl von Ketten (Chains) erstellt (im Gegensatz zu Blockchain, wo es typischerweise genau eine Kette gibt). Die Blöcke der Kette bilden eine Datensammlung. Die verteilte Speicherstruktur kann als modifizierte Blockchain Struktur ausgebildet sein. Die Modifikation bezieht sich darauf, dass ein Datenblock gemäß einer grundlegenden Ausführungsform der Erfindung lediglich aus dem berechneten Dokumentationswert (Hashwert) besteht, der eine einzigartige Signatur darstellt. Ein Datenblock muss keinen Verweis auf die anderen Blöcke der Kette enthalten. Um einen Datenblock auszurechnen muss, im Unterschied zur klassischen Blockchain, nicht auf einen Miningpool zurückgegriffen werden. Die Berechnungen können deutlich schneller und auch auf weniger leistungsfähigen Computereinheiten ausgeführt werden und sind dennoch fälschungssicher hinterlegt. Ein weiterer struktureller Unterschied zur Architektur der Blockchain liegt darin, dass die modifizierte Blockchain Struktur mehrere Ketten umfasst und somit einen Multi-Ketten Datenraum darstellt. Dabei kann sich eine Kette in einer ersten Ausführungsform jeweils auf einen Verknüpfungspartner, z.B. einen Patienten oder eine Umkehrosmoseanlage beziehen. Es können mehrere Ketten (z.B. eine Kette pro Gebrauchsgegenstand oder pro Patient) in einer verteilten Speicherstruktur (z.B. auf einer Gruppe von Dialysegeräten) gespeichert sein. Da ein Patient einerseits seinen Identifikator in der Regel nicht ändert, aber andererseits viele Verbrauchsgüter (die jeweils individuelle Identifikatoren aufweisen) verwendet, sollten viele Ketten pro Patient entstehen - aber nur eine pro Gebrauchsgegenstand.

In einer bevorzugten Ausführungsform der Erfindung wird auf einem Dialysegerät ein Speicher oder ein Speicherbereich bereitgestellt, in dem eine Instanz der verteilten Speicherstruktur abgelegt ist (z.B. als modifizierte Blockchain). Dort können alle auf dem Dialysegerät erfolgten (also validierten) und/oder intendierten (noch nicht validierten) Verknüpfungsereignisse gespeichert werden. Die Verknüpfungsereignisse können sich auf unterschiedliche Verbrauchsgegenstände und/oder auf unterschiedliche Patienten beziehen. Dies hat den technischen Vorteil, dass eine Absicherung mittels einer Validierungsprüfung lokal auf einem Teilnehmer(gerät) ausgeführt werden kann. Insbesondere kann die Validierungsprüfung vorteilhaftweise auch dann ausgeführt werden, wenn zeitweise keine (oder keine ausreichende) Kommunikationsverbindung zu anderen Geräten besteht, da die Speicher der Geräte stets synchronisiert sind.

In einer Ausführungsform der Erfindung umfasst die Berechnung des Dokumentationswertes das Berechnen eines Wiederholungswertes. Der Wiederholungswert repräsentiert dabei eine Wiederholungsrate oder eine zulässige Anzahl von Wiederholungen für ein Verknüpfungsereignis zwischen dem ersten und zweiten Verknüpfungspartner auf eindeutige Weise. Damit kann auf sichere Weise nachvollzogen und differenzierbar dokumentiert werden, ob und wenn ja, wie viele Wiederholungen von Verknüpfungsereignissen bereits erfasst worden sind. In einer Konfigurationsphase ist es z.B. konfigurierbar, wie viele Wiederholungen eines Verknüpfungsereignisses als zulässig erachtet werden. So kann es in einigen Anwendungen z.B. erlaubt werden, dass ein Satz von Disposables für einen bestimmten Patienten von ihm mehr als einmal verwendet werden darf. Allerdings kann eine konfigurierbare maximale Anzahl von Wiederholungen definiert worden sein, um eine darüber hinaus gehende Verwendung zu beschränken. Der große Vorteil ist darin zu sehen, dass aufgrund der verteilten Speicherstruktur gewährleistet werden kann, dass der Patient ein initial mit ihm verknüpftes/ihm eindeutig zugewiesenes Disposable mit unterschiedlichen Dialysemaschinen verwenden kann.

In einer weiteren Ausführungsform der Erfindung erfolgt die Berechnung des Dokumentationswertes durch Anwenden einer (z.B. kryptologischen) dritten Rechenvorschrift auf ein erstes Teilergebnis und ein zweites Teilergebnis zur Bereitstellung eines dritten Teilergebnisses. Das erste Teilergebnis wird berechnet durch Anwenden der ersten Rechenvorschrift auf den ersten Identifikator. Das zweite Teilergebnis wird berechnet aus Anwendung der zweiten Rechenvorschrift auf den zweiten Identifikator. In einer vorteilhaften Variante kann auf das dritte Teilergebnis gegebenenfalls eine weitere (mathematische) Verknüpfungsfunktion angewendet werden, um das Gesamtergebnis zu berechnen. Andernfalls kann das dritte Teilergebnis auch als Gesamtergebnis fungieren. Bei Wahl von geeigneten Verknüpfungsfunktionen und/oder Rechenvorschriften ist das Verfahren und System nicht oder nur durch sehr lange dauernde, aufwändige Rechenverfahren korrumpierbar.

In einer weiteren bevorzugten Ausführungsform der Erfindung erfolgt bei nicht erfolgreicher Validierung automatisch eine Sperrung des Gerätes zumindest für die intendierte Anwendung für die jeweiligen Verknüpfungspartner. Dies hat den Vorteil, dass das Gerät somit für andere autorisierte Verknüpfungspartner weiter betrieben werden kann. Die Sperrung kann mit einem Warnhinweis gekennzeichnet sein, der auf einer Benutzeroberfläche ausgegeben wird. Damit kann die Sicherheit des Gerätebetriebs erhöht werden. Durch die automatische Sperrung des Gerätes können Fehler durch eine manuell unterlassene Prüfung vermieden und ausgeschlossen werden.

Vorzugsweise wenden alle Geräte dieselben Rechenvorschriften zur Berechnung des Dokumentationswertes an, wenn sie auf die verteilte Speicherstruktur zugreifen.

Gemäß einer vorteilhaften Weiterbildung der Erfindung kann das Validieren und/oder das Autorisieren des Verknüpfungsereignisses zeitlich getaktet und/oder ereignisbasiert ausgeführt werden. Dies hat den technischen Hintergrund, dass es für bestimmte Typen von Disposables notwendig ist, zu überwachen, wie lange (in welchen Zeitintervallen) und/oder in welcher Menge sie verwendet worden sind. Dies ist insbesondere bei der Überwachung der Verwendung eines Adsorbers bei einer therapeutischen Apherese wichtig. Das Erfassen eines Verknüpfungsereignisses erfolgt nach einem vorkonfigurierbaren Schema (z.B. Zeitschema). So wird es möglich, eine zeitliche Taktung von Verknüpfungsereignissen zu erzeugen, um eine Betriebs- und/oder Standzeit überprüfbar zu machen und manipulationssicher zu speichern.

Vorstehend wurde die Lösung der Aufgabe anhand des Verfahrens beschrieben. Dabei erwähnte Merkmale, Vorteile oder alternative Ausführungsformen sind auch auf die anderen beanspruchten Gegenstände zu übertragen und umgekehrt. Mit anderen Worten können auch die gegenständlichen Ansprüche (die beispielsweise auf ein System oder auf ein Gerät oder ein Absicherungsmodul gerichtet sind) die Merkmale umfassen, die im Zusammenhang mit dem Verfahren beschrieben und/oder beansprucht sind und umgekehrt. Die entsprechenden funktionalen Merkmale des Verfahrens werden dabei durch entsprechende gegenständliche Module, insbesondere durch Hardware-Module oder Mikroprozessor-Module, des Systems bzw. des Produktes ausgebildet und umgekehrt.

Gemäß einem weiteren Aspekt bezieht sich die Erfindung auf ein Computerprogramm mit Programmcode, der für das Ausführen des vorstehend beschriebenen Absicherungsverfahrens geeignet ist, wenn das Computerprogramm auf einem Computer oder einer Computer-basierten Verarbeitungseinheit oder einem Absicherungsmodul ausgeführt wird. Das Computerprogramm kann auf einem Speicher gespeichert sein und z.B. als App auf dem Dialysegerät und/oder der Umkehrosmoseanlage verfügbar sein. Es kann aber auch auf einer zentralen Recheneinheit implementiert sein, die mit den jeweiligen Geräten in Datenaustausch (Kommunikationsverbindung) steht. Das Programm kann auch via Download über eine Netzwerkverbindung von einer computer-basierten Einheit geladen werden.

In einem weiteren Aspekt bezieht sich die Erfindung auf ein Absicherungsmodul für ein medizinisches Gerät, auf das eine Applikation geladen und ausgeführt werden kann, um ein Verfahren gemäß den vorangehenden Verfahrensansprüchen auszuführen, wenn die Applikation auf einer digitalen Verarbeitungseinheit z.B. auf dem Absicherungsmodul ausgeführt wird. Dazu umfasst das Absicherungsmodul:

- eine Einleseschnittstelle zum Einlesen eines ersten und eines zweiten Identifikators;

- eine elektronische Verarbeitungseinheit, die ausgebildet ist, ein Absicherungsverfahren wie vorstehend beschrieben auszuführen;

- einen Speicher zur Speicherung eines berechneten Dokumentationswertes; alternativ kann der Speicher ausgelagert sein, so dass auf dem Absicherungsmodul nur eine Schnittstelle zu dem Speicher integriert ist;

- einer Schnittstelle zu dem Netzwerk, über das die Geräte in Datenaustausch stehen.

Dieser Aspekt der Erfindung zielt auf dieselbe Lösung der Aufgabe ab, allerdings wird das oben beschriebene Verfahren hier nicht direkt auf dem Gerät (Dialysegerät oder Umkehrosmoseanlage) ausgeführt, sondern auf einer separaten digitalen computer-basierten Einheit, nämlich dem Absicherungsmodul, das mit dem Gerät in Datenaustausch steht und zur zusätzlichen Absicherung durch die Ausführung des Verfahrens dient. Das Absicherungsmodul kann dann - je nach Ergebnis des Absicherungsverfahrens - ein Freigabe- oder ein Sperrsignal an das Gerät senden. Das Absicherungsmodul kann z.B. als mobiles elektronisches Endgerät (Tablet, Smartphone etc.) ausgebildet sein. Das Absicherungsmodul kann auch auf dem Dialysegerät oder einem anderen Gerät implementiert sein. Das Absicherungsmodul kann als Softwaremodul bereitgestellt werden und kann Teil einer virtuellen Maschine sein.

In einer Ausführungsform der Erfindung weist eine das Absicherungsmodul umfassende Einheit oder das Absicherungsmodul selbst eine Schnittstelle zu weiteren Absicherungsmodulen von anderen Geräten des Netzwerkes auf, um insgesamt mit den jeweils zugeordneten Speichern eine verteilte Speicherstruktur zu bilden, die z.B. als DLT-Struktur ausgebildet sein kann.

In einem weiteren Aspekt bezieht sich die Erfindung auf ein medizinisches Gerät mit einem Absicherungsmodul, wie vorstehend beschrieben. In diesem Fall ist das Absicherungsmodul als reine elektronische Verarbeitungseinheit ohne weitere Funktionalität implementiert, wie z.B. ein Mikroprozessor oder eine integrierte Schaltkreisanordnung (FPGA etc.).

In einem weiteren Aspekt bezieht sich die Erfindung auf ein System zur Absicherung eines Gerätes im medizinischen Umfeld vor einem nicht autorisierten Betrieb des Gerätes, wobei die Autorisierung abhängig ist von einem Verknüpfungsereignis zwischen zwei Verknüpfungspartnern im Rahmen eines Gerätebetriebs, wobei alle oder ausgewählte Verknüpfungspartner auf eineindeutige Weise einem Identifikator zugeordnet sind. Einer der Verknüpfungspartner ist ein Gebrauchsgegenstand, der bei dem Gerätebetrieb verwendet wird. Ein wichtiger Aspekt ist darin zu sehen, dass das Gerät in einem verteilten Geräteverbund betrieben wird und dass die Absicherung eines dieser Geräte des Geräteverbundes ohne zentrale Steuerungsinstanz ausführbar sein soll. Dazu umfasst jedes Gerät ein Absicherungsmodul, wie vorstehend beschrieben, das mit einer verteilten Speicherstruktur über das Netzwerk in Datenaustausch steht.

In einer Ausführungsform der Erfindung bilden die Speicher der Geräte eine verteilte Datenstruktur (insbesondere eine distributed ledger), die allerdings kontinuierlich synchronisiert ist, so dass ein lokaler Zugriff auf den Speicher des lokalen Gerätes ausreicht, um einen Dokumentationswert zu validieren. Lokal liegen zumindest und vor allem die synchronisierten Datensätze vor, die mit einer gewissen Mindestwahrscheinlichkeit an diesem Gerät (innerhalb der klinischen Einrichtung) benötigt werden, wenn man davon ausgeht, dass der Patient in der Regel dieselbe Klinik besucht. Damit können sowohl die Effizienz des Verfahrens gesteigert und die erforderlichen (Netzwerk-) Ressourcen geschont werden. Nicht lokal vorliegende Sätze können über das Netzwerk auch adressiert werden. Gegebenenfalls wird dafür - je nach verfügbarer Bandreite - etwas länger für die Validierung zur anschließenden Autorisierung benötigt. Wenn der Speicher eines Gerätes begrenzt ist, dass nur ein Teil der distributed ledger Struktur lokal gespeichert werden soll, dann könnte man vorteilhafterweise den lokal vorgehaltenen Teil so auswählen, dass er Patienten betrifft, bei denen die Wahrscheinlichkeit, dass sie erneut dort zur Behandlung sein werden, erhöht ist. Eine erhöhte Wahrscheinlichkeit, wieder dort zu behandeln, könnte beispielsweise automatisch aus einem Datensatz abgelesen werden, dass ein Patient bereits dort behandelt wurde. Dadurch ergibt sich den Vorteil, dass ein Autorisierungsverfahren durch Abgleich mit dem lokal vorgehaltenen Teil des Ledger möglich wäre. Das wäre wiederum schneller, als ggf. Ledger-Teile von fernen und langsam angekoppelten Geräten herunterzuladen. In Varianten, bei denen jedes Gerät nur einen Ausschnitt des Ledgers vorhält, wäre diese Variante des Verfahrens besonders vorteilhaft.

In einer Ausführungsform der Erfindung kann eine Kennzeichnung eines ersten Verknüpfungspartners (z.B. medizinisches Disposables) eine elektronische Verarbeitungseinheit (z.B. ein RFID Chip) sein, wobei die elektronische Verarbeitungseinheit dazu eingerichtet ist, eine eindeutige Disposable- Kennzeichnung zu speichern, die wiederum auf eineindeutige Weise einem Disposable-Identifikator zugeordnet ist. Ein zweiter Verknüpfungspartner kann ein Patient sein, der eine Dialysebehandlung mit dem Disposable an einem Gerät durchführen möchte. Der Patient hat einen ihn identifizierenden Patientenidentifikationscode (z.B. eine ID-Karte des Patienten, z.B. eine Gesundheitskarte, mit der er sich an dem Dialysegerät anmeldet). Das Verknüpfungsereignis repräsentiert die Verwendung des jeweiligen Disposables für den jeweiligen Patienten bei einer Behandlung an einem bestimmten medizinischen Gerät.

In einer weiteren, vorteilhaften Ausführungsform der Erfindung ist ein erster Verknüpfungspartner eine Membran, die mit einem Identifikationshinweis gekennzeichnet ist. Die Kennzeichnung kann z.B. in Form eines integrierten oder aufgebrachten RFID-Chips, der als elektronische Verarbeitungseinheit fungiert, erfolgen. Die Kennzeichnung kann über geeignete Einlesemittel eingelesen werden (Scanner) und ist auf eineindeutige Weise einem Membran-Identifikator (als digitaler Datensatz) zugeordnet. Ein zweiter Verknüpfungspartner ist eine Umkehrosmose-Anlage, die ebenfalls eineindeutig über einen Anlagen-Identifikator identifizierbar ist. Das Verknüpfungsereignis repräsentiert die Verwendung der Membran in der Umkehrosmose-Anlage.

Eine weitere Aufgabenlösung sieht ein Computerprogrammprodukt vor, mit Computerprogrammcode zur Durchführung aller Verfahrensschritte des oben näher beschriebenen Verfahrens, wenn das Computerprogramm auf einem Computer ausgeführt wird. Dabei ist es auch möglich, dass das Computerprogramm auf einem von einem Computer lesbaren Medium gespeichert ist. Das Computerprogrammprodukt kann z.B. als gespeicherte, ausführbare Datei, ggf. mit weiteren Bestandteilen (wie Libraries, Treibern etc.) oder als Computer mit dem bereits installierten Computerprogramm ausgebildet sein.

Kurze Übersicht über die Figuren

In der folgenden detaillierten Figurenbeschreibung werden nicht einschränkend zu verstehende Ausführungsbeispiele mit deren Merkmalen und weiteren Vorteilen anhand der Figuren der Zeichnung erläutert. In dieser zeigen:

Fig. 1 eine schematische Übersicht über eine verteilte Speicherstruktur gemäß einer ersten Ausführungsform der Erfindung mit mehreren Dialysegeräten, an denen unterschiedliche Patienten behandelt werden können;

Fig. 2 eine schematische Übersicht über eine verteilte Speicherstruktur gemäß einer zweiten Ausführungsform der Erfindung mit mehreren Umkehrosmoseanlagen, die mit unterschiedlichen Membranen betrieben werden können;

Fig. 3 eine schematische Darstellung zur Interaktion und zum Nachrichtenaustausch zwischen den Geräten und der verteilten Speicherstruktur; und

Fig. 4 ein Ablaufdiagramm eines Verfahrens gemäß einer bevorzugten Ausführungsform der Erfindung.

Detaillierte Beschreibung der Figuren

Die Erfindung dient dazu, die Sicherheit des Gerätebetriebs von Dialysegeräten DG, die mit Verbrauchsgegenständen, wie Einwegartikeln (z.B. Dialysatoren) betrieben werden müssen, oder anderen medizinischen Geräten, wie z.B. Umkehrosmoseanlagen, die mit Membranen betrieben werden, zu erhöhen.

In einer ersten Ausführungsform der Erfindung bezieht sich ein erster Verknüpfungspartner auf einen Patienten, der über einen Code oder Identifikator eines Identifikationsmittels, wie z.B. eine Patienten-Karte, eindeutig identifizierbar ist und sich mit seinem Identifikator an dem Dialysegerät anmeldet. Ein für die Behandlung des Patienten vorgesehenes Dialyseset stellt einen zweiten Verknüpfungspartner dar. Hintergrund dieser Ausführungsform ist, dass der Gebrauchsgegenstand, z.B. in Form eines Dialysators/ Schlauchsets, dem Patienten zur eigenverantwortlichen Verwendung zur Verfügung gestellt wird und sich der Patient dann mit diesem Set an einer beliebigen Dialysemaschine seiner Wahl anmeldet, um die Behandlung durchzuführen. Je nach Anwendungsfall soll der Gebrauchsgegenstand nur einmalig oder nur mit einer begrenzten Anzahl von Wiederholungen, aber auf jeden Fall nur für ein und denselben Patienten verwendet werden, beispielsweise um eine Übertragung von Krankheitserregern zwischen zwei Patienten zu verhindern, die u. U. auch durch eine Reinigung oder Desinfektion des begrenzt wiederverwendbaren Disposables nicht vollständig beseitigt werden. Die Erfindung löst das Problem, indem ein Dokumentationsverfahren bereitgestellt wird, das so erweitert ist, dass Wiederholungen von Verknüpfungsereignissen fälschungssicher und zudem anonym dokumentiert und/oder überprüft werden können und, dass überprüft werden kann, ob ein bestimmter Verknüpfungspartner bereits ein Verknüpfungsereignis erfahren hat. Zudem kann sichergestellt werden, dass bereits verknüpfte Partner nicht anderweitig verknüpft werden können. Aus Gründen der Infektionsgefahr darf der Gebrauchsgegenstand keinesfalls von einem Patienten zu einem anderen wechseln. Die Erfindung schafft ein System, mit dem dies automatisch sichergestellt werden kann. Dies wird insbesondere gewährleistet durch ein Gerät im medizintechnischen Kontext, in dem das Disposable verwendet werden soll. Das Gerät prüft selbstständig und automatisch die Zulässigkeit einer Verknüpfung der Verknüpfungspartner und kann ggf. eine Behandlung unterbinden oder einschränken.

In einer zweiten Ausführungsform der Erfindung bezieht sich der erste Verknüpfungspartner auf eine Membran, die in einer Umkehrosmoseanlage (zweiter Verknüpfungspartner) betrieben werden soll. Die Membran ist gekennzeichnet und z.B. mit einer elektronischen Kennzeichnung über einen RFID-Chip eindeutig identifizierbar. Die Kennzeichnung kann von einem z.B. auf der RO-Anlage ausgebildeten Lesegerät ausgelesen und in Form eines digitalen Identifikators zur weiteren Verarbeitung bereitgestellt werden. Die Membran meldet sich sozusagen mit diesem Identifikator an der Umkehrosmoseanlage an. Bei dieser Ausführungsform gilt es zu überwachen, dass die Umkehrosmoseanlage stets auf zulässige Weise betrieben wird und die Verbrauchsgegenstände (z.B. Membran) dabei nicht über den vorgesehenen Verwendungszeitraum hinaus verwendet werden. Das hier vorgeschlagene Dokumentationsverfahren ist deshalb derart erweitert, dass eine bestimmte Anzahl von identischen Verknüpfungsereignissen ermöglicht werden kann, aber alle weiteren Verknüpfungsereignisse zu verhindern. Wenn z.B. die osmotische Membran länger als vorgesehen eingesetzt wird, kann die Wasserqualität nicht mehr gewährleistet werden und die Dialysemaschine, die über die Umkehrosmoseanlage versorgt wird, kann nicht mehr gemäß den Sicherheitsstandards betrieben werden. Die osmotische Membran, die in der Umkehrosmoseanlage nur die Trägerflüssigkeit (Solvent) durchlässt und die gelösten Stoffe (Solute) zurückhält, muss diesen hohen Drücken standhalten können. Wenn der Druckunterschied das osmotische Gefälle mehr als ausgleicht, passen die Solventmoleküle wie bei einem Filter durch die Membran, während die "Verunreinigungsmoleküle" zurückgehalten werden. Die Membrane sind daher sehr sensibel. Um Beschädigungen der Membran zu verhindern, können Filter vorgeschaltet werden. Ein Feinfilter kann mechanische, ein Aktivkohlefilter chemische Beschädigungen (z. B. durch Chlor) verhindern. Grundsätzlich zielt die hier vorgeschlagene Lösung darauf ab, ein Ereignis zu dokumentieren, das eine Verknüpfung von zwei Verknüpfungspartnern (z.B. Gebrauchsgegenstand/Disposable und Patient, der bei seiner Dialysebehandlung das Disposable verwendet) darstellt. Konkret kann ein solches Verknüpfungsereignis beispielsweise den Fall repräsentieren, dass eine Person einen Gebrauchsgegenstand, etwa einen Dialysator, bei einer Behandlung benutzt (Verbrauchsgegenstände werden in dem Zusammenhang auch "Disposable" genannt.).

In einer Weiterbildung kann nicht nur der Betrieb des Gerätes dokumentiert werden, sondern das Gerät kann auch gegen eine unautorisierte Verwendung von Verbrauchsgegenständen auf dem Gerät abgesichert werden. Wenn ein intendiertes Verknüpfungsereignis von zwei Verknüpfungspartnern als nicht autorisiert ausgewertet worden ist, dann kann das Gerät zumindest für genau diese Verwendung mit den beiden Verknüpfungspartnern gesperrt werden. In Notfällen ist jedoch eine manuelle Sondergenehmigung möglich, indem ein manuelles Überschreiben erfolgt. Dazu kann die Eingabe eines vordefinierten Freigabecodes auf dem Gerät notwendig sein.

Es wird vorausgesetzt, dass die jeweiligen Verknüpfungspartner über eine/n ihnen jeweils zugeordnete/n Kennzeichnung bzw. einen Identifikator eindeutig identifiziert werden können. So kann sich der Patient z.B. über seine Patientenkarte, und der vom Patienten mitgeführte Dialysator über einen unlösbar damit verbundenen RFID-Chip identifizieren. Ebenso kann die Membran über einen unlösbar daran angebrachten RFID-Chip identifizierbar sein.

Das Verknüpfungsereignis wird dokumentiert, indem

a) eine erste mathematische Funktion f auf den ersten Identifikator (z.B. ID-D) angewendet wird: f(ID-D)

b) eine zweite mathematische Funktion g auf den zweiten Identifikator (z.B. ID-P) angewendet wird: g(ID-P)

c) f(ID-D) und g(ID-P) mathematisch (durch eine Anwendung einer weiteren Rechenvorschrift $\varphi$ (RV3, kurz bezeichnet mit $\varphi$) verknüpft werden: f(ID-D) $\varphi$ g(ID-P), um einen Dokumentationswert W zu berechnen. Durch die Anwendung der Funktionen fließen die Identifikatoren der Verknüpfungspartner in die Dokumentation ein.

d) In einer allgemeineren Form wird der Dokumentationswert um einen Parameter k erweitert:

$$W = f(ID\text{-}D)\ \varphi\ g(ID\text{-}P)\ \varphi\ k$$

[mit k=1 ergibt sich dann der Fall aus c)]

e) Der berechnete Dokumentationswert W wird in einen Speicher MEM geschrieben.

Die erste und die zweite mathematische Funktion können identisch sein.

Im einfachsten Fall ist f eine Multiplikation mit 1, dann ist f(ID-D) = ID-D. In einem bevorzugten Fall ist f eine kryptologische Funktion oder eine Hash-Funktion (auch Streuwertfunktion), wie z.B. SHA, SHA-1, MD5. Andere Funktionen sind vorstellbar.

Dasselbe gilt analog für g.

Dasselbe gilt analog auch für die weitere Rechenvorschrift RV3 $\varphi$.

In einem bevorzugten Ausführungsbeispiel sind f, g, $\varphi$ Hashfunktionen, so dass der erzeugte Dokumentationswert eine fest definierte Länge hat und quasi einen Fingerabdruck der Identifikatoren repräsentiert. Der dann entstehende Dokumentationswert W = f(ID-D) $\varphi$ g(ID-P) $\varphi$ k ist kryptologisch wertvoll und nicht einfach vorherzusehen oder auf die ausgangswerte ID-D und ID-P zurückzuführen. Dies hat den Vorteil, dass die tatsächliche ID (ID-D oder ID-P) in dem Datensatz nicht im Klartext erkennbar wäre, was erhöhten Ansprüchen an die Wahrung der Privatsphäre entgegenkommt und auch die Fälschung von Seriennummern der Disposables erschwert.

In einem bevorzugten Ausführungsbeispiel sind die Identifikatoren ID-D und ID-P einzigartig und in einer schwer oder nicht vorhersehbaren Weise erzeugt, z.B. unter Verwendung eines Zufallsgenerators.

Wenn man nun eine Vielzahl von Disposables D_1 bis D_1000 hat, haben all diese Disposables unterschiedliche, nicht zu erratende oder vorhersagbare, eindeutige Identifikatoren ID-D_1 bis ID-D_1000.

Wenn man nun außerdem eine Vielzahl von Personen P_1 bis P_1000 unterschiedliche, nicht zu erratende oder vorhersagbare, eindeutige Identifikatoren ID-P_1 bis ID-P_1000 zuordnet, ist man in der Ausgangslage für das letztgenannte bevorzugte Ausführungsbeispiel.

Für jede beliebige Kombination von Verknüpfungspartnern (D_i, P_j) entsteht mittels dieser Idee ein eindeutiger, kryptologisch sicherer, nicht vorhersehbarer Dokumentationswert W = f(ID-D) $\varphi$ g(ID-P) $\varphi$ k. Durch die Auswahl geeigneter kryptologischer Funktionen für f, g und $\varphi$ kann ein Rückrechnen auf die Ursprungswerte beliebig schwierig bis praktisch unmöglich gemacht werden. Die berechneten Dokumentationswerte W stellen also ein eindeutiges Muster oder eine eindeutige Signatur der bei dem Verknüpfungsereignis beteiligten Verknüpfungspartner dar.

Ein Angreifer, der einen Identifikator nicht kennt, wird viel Aufwand betreiben müssen, um eine sinnvolle Kombination zu erraten, da die Identifikatoren nicht leicht erratbar sind. Ein Gerät, das lokal die Identifikatoren zweier Verknüpfungspartner einlesen kann, hat es dagegen leicht. Dies hat den technischen Vorteil, dass ein Lesezugriff auf eine verteilte Speicherstruktur DL, sehr effizient ausgeführt werden kann.

Zur Speicherung der berechneten Dokumentationswerte W / Muster:
Diese Dokumentationswerte W (im Folgenden auch kurz: Werte) müssen nach ihrer Berechnung jeweils gespeichert werden. Das kann im einfachsten Fall einfach der Wert selbst sein. In erweiterten Varianten können auch noch Zeitstempel oder weitere Daten mit dem Muster gespeichert werden.

Alle Werte W eines bestimmten Verfahrens werden in einer gemeinsamen Datenstruktur gespeichert, welche als distributed Ledger DL eingerichtet ist.

In einer bevorzugten Variante sind die Eigenschaften der Identifikatoren (ID-D, ID-P) und der Funktionen f, g , $\varphi$ so gewählt, dass alle mit dem Verfahren erzeugbaren Werte eine gleichbleibende Größe und/oder ein gleichbleibendes Format haben. Dadurch ergibt sich eine Speicherung mit gleichgroßen Blöcken. Optional kann ein Padding (Auffüllen) mit Nullen oder Einsen auf die einheitliche Blockgröße erforderlich sein.

In einer bevorzugten Variante gibt es Speicherblöcke, in denen jeweils ein einzelner Wert W abgelegt wird.

Die Speicherung kann lokal in einem Endgerät stattfinden, das das Verfahren ausführt (z.B. Dialysegerät, Umkehrosmoseanlage, mobiles Endgerät eines Anwenders). Die Speicherung kann remote in der Cloud, auf einem Server oder in einem Backend stattfinden, wobei im letzten Fall ein lokales Gateway-Gerät benötigt wird. Bevorzugt findet die Speicherung so statt, dass alle beteiligten Geräte die Muster direkt nach der Erzeugung lokal speichern und danach an im Netzwerk verbundene Geräte verteilen. Dieser Vorgang wird als "synchronisiertes Speichern" bezeichnet.

Bevorzugt gibt es eine Vielzahl von in einem Netzwerk verbundenen Geräten, die das Verfahren ausführen und z.B. über das Internet mithilfe z.B. einer Peer-to-Peer-Kommunikation inkrementell ihre Speicher so synchronisieren, dass nach einer gewissen Zeit alle erzeugten Werte i, in der lokalen Distributed Ledger DL aller Geräte vorliegen. Ein derart aufgestelltes Netzwerk benötigt keine zentralen Instanzen.

Alternativ kann die Verteilung der Muster auf die im Netzwerk verbundenen Geräte mittels eines Algorithmus so gesteuert sein, dass nicht jedes Gerät alle Muster speichert, aber alle Muster innerhalb einer definierten Gruppe von kommunikativ verbundenen Geräten zumindest einmal, vorzugsweise redundant gespeichert sind. Die Verteilung der Muster zur Speicherung kann ohne dedizierten zentralen Verwaltungs-Master nach grundsätzlich bekannten Verfahren erfolgen. Dabei kann vorzugsweise eine Speicherung wiederholt in Zusammenhang mit einem Gerät verwendeter Identifikatoren lokal in diesem Gerät erfolgen, um den Netzwerkverkehr zu reduzieren.

Bevorzug wird dabei eine Distributed-Ledger-Technologie verwendet, um Übereinkunft über alle durch die Werte W dokumentierten Verknüpfungsereignisse innerhalb des Gerätenetzwerks zu erreichen und diese Werte W im Netzwerk zu aktualisieren. So ergibt sich eine dezentrale Verknüpfungsereignisdatenbank.

Bevorzugt gibt es einen Konsens-Mechanismus zur Gewährleistung identischer Kopien des Distributed Ledger DL auf allen beteiligten Instanzen.

Verarbeitung der gespeicherten Muster:

Wenn man ein Verknüpfungsereignis (wie z.B. eine Dialysebehandlung) herbeiführen möchte und die Identifikatoren beider Verknüpfungspartner kennt (ID-D, ID-P) kann man mit einem Gerät das Verfahren ausführen und erhält das Muster bzw. den berechneten Dokumentationswert W zu diesen Partnern. Nun kann der Distributed Ledger DL durchsucht werden, ob darin genau dieser Wert W gespeichert ist. Dabei ist es entscheidend, dass der Wert nicht lokal, sondern auf einer anderen Instanz der Distributed Ledger DL, z.B. auf einem anderen Gerät, gespeichert sein kann und dies auch automatisch durch die Abfrage der Distributed Ledger DL erfasst wird.

Wenn man den gesuchten Wert W nicht auffindet, bedeutet das, dass noch kein Verknüpfungsereignis dieser beiden Verknüpfungspartner dokumentiert ist. In einer einfachen Ausführungsform der Erfindung gilt der Wert damit als validiert.

Wenn man den gesuchten Wert W auffindet, bedeutet das in einer bevorzugten Ausführungsform, dass es bereits ein Verknüpfungsereignis dieser beiden Verknüpfungspartner gegeben hat. In einer Variante kann das dazu führen, dass kein weiteres Verknüpfungsereignis zugelassen wird und der Wert nicht validiert wird. Der Gerätebetrieb kann in Antwort auf die fehlende Autorisierung automatisch gesperrt werden.

Die Anforderungen an die Geräte, die das erfindungsgemäße Verfahren ausführen, sind:

- Sie müssen in Kenntnis von ID-D gelangen können (durch manuelle Eingabe, Scanner für Barcode oder QR-Code, RFID-Leser, Bildererkennung, Datenempfang)
- Sie müssen in Kenntnis von ID-P gelangen können (durch einen Patientenkartenleser und die vorstehend beschriebenen Methoden)
- Sie müssen eine Kommunikationsverbindung haben.
- Sie müssen einen Speicher haben.
- Die kryptografischen Berechnungen können lokal oder remote erfolgen. Wenn sie lokal erfolgen soll, muss das Gerät natürlich in der Lage zur Berechnung sein, d.h. die Berechnungsvorschrift kennen und ausreichend Prozessor- und Speicherressourcen aufweisen.
- Die Internetanbindung kann direkt oder über Zwischeninstanzen (wie z.B. lokales WiFi) erfolgen.

Die Geräte können zum Beispiel sein: eine Behandlungsmaschine (Dialysegerät DG), ein Smartphone und/oder ein Tablet als Gateway, das eine Behandlung freigibt.

Wiederholungen von Verknüpfungsereignissen dokumentieren:

Mit dem grundlegenden Verfahren sehen die Werte W von zwei Verknüpfungsereignissen derselben Verknüpfungspartner gleich aus, denn für beide Ereignisse würde die Berechnung f(ID-D) φ g(ID-P) φ k = W lauten.

Daher wird das Verfahren erweitert, um solche Wiederholungen unterscheidbar zu machen. Wenn die Wiederholungen unterscheidbar sind, können sie dokumentiert werden und die Anzahl von Wiederholungen kann ermittelt werden.

Mittels Blockchain-Verfahren könnte man dieses Problem lösen, indem man einen Wert W(n+1) ausrechnet, indem man den Wert W(n) mit verhasht. Das wäre dann also

$$W(1) = f(ID\text{-}D) \; \varphi \; g(ID\text{-}P)$$

$$W(2) = W(1) \; \varphi \; f(ID\text{-}D) \; \varphi \; g(ID\text{-}P)$$

$$W(3) = W(2) \; \varphi \; f(ID\text{-}D) \; \varphi \; g(ID\text{-}P) = W(1) \; \varphi \; f(ID\text{-}D) \; \varphi \; g(ID\text{-}P) \; \varphi \; f(ID\text{-}D) \; \varphi \; g(ID\text{-}P)$$

$$W(n+1) = W(n) \; \varphi \; f(ID\text{-}D) \; \varphi \; g(ID\text{-}P) = n+1 \; \text{mal ausgeführt} \; f(ID\text{-}D) \; \varphi \; g(ID\text{-}P).$$

Das Verfahren könnte zwar kryptologisch machbar und unterscheidbar sein, aber es würde extrem aufwändig, insbesondere dahingehend, im Voraus zu überprüfen, welche Eingangswerte sich dafür eignen und welche zu uneindeutigen Ergebnissen ("Kollisionen") oder Abbrüchen führen. Außerdem bietet es keine Abkürzung zur Errechnung, wie viele Wiederholungen bereits stattgefunden haben. Man müsste brute force mäßig so lange die Hashfunktion wiederholen, bis man kein vergleichbares Ergebnis im Distributed Ledger mehr findet.

Daher schlägt die vorliegende Erfindung ein anderes Konzept vor. Dazu wird als Parameter k ein dritter Identifikator ID-n eingeführt, welcher die Wiederholung kennzeichnet. Im einfachsten Fall ist ID-n einfach die natürliche Zahl n selbst, die der Wiederholung eines Verknüpfungsereignisses entspricht.

Es ist aber auch denkbar, dass es z.B. eine Tabelle gibt, die jedem n einen Identifikator ID-n zuordnet. Hierbei ist wichtig, dass alle an dem Verfahren beteiligten Instanzen auf die gleiche Weise einem Wiederholungswert n einen Identifikator ID-n zuordnen. In anderen Worten: Es muss vorhersagbar sein, damit alle beteiligten Geräte und Instanzen sich ohne Eingriff von außen von dem Wert einer Wiederholung zum nächsten "hangeln" können.

Die so ausgerechneten Werte W = f(ID-D) φ g(ID-P) φ k sind dann entweder

  a) Mit k = ID-(n+1):

$$W(n+1) = f(ID\text{-}D) \; \varphi \; g(ID\text{-}P) \; \varphi \; ID\text{-}(n+1)$$

  b) Mit k = h (ID-(n+1)):
  W(n+1) = f(ID-D) φ g(ID-P) φ h (ID-(n+1)), wobei h eine kryptologische Funktion ähnlich wie f und g sein könnte.
  c) Oder, falls doch eine erhöhte Sicherheitskette der Dokumentation der Kette erwünscht ist (wie bei Blockchain)
  W(n+1) = W(n) φ f(ID-D) φ g(ID-P) φ h (ID-(n+1)).

Bei Wahl von geeigneten Werten für ID-n kann so sichergestellt werden, dass die Werte W zu verschiedenen Wiederholungen (d.h. mit verschiedenen Wiederholungszahlen n) verschieden sind.

In den Varianten a) und b) müsste man auch nicht die gesamte Reihe von Werten zu verschiedenen Wiederholungen berechnen, um einen neuen Wert W zur aktuellen Wiederholung zu errechnen.

Wenn ein Gerät nicht weiß, wie viele Wiederholungen durchgeführt wurden, und das ermitteln möchte, kann es nacheinander verschiedene Werte W zu den vorliegenden Verknüpfungspartnern ausrechnen und den Distributed Ledger DL nach den Werten W durchsuchen. Der bei der Durchsuchung gefundene Wert W zu der höchsten Wiederholungszahl n entspricht der Anzahl bisher erfolgter und dokumentierter Wiederholungen.

Überprüfen, ob ein Verknüpfungspartner bereits verknüpft ist:

Ein praktischer Fall für die Anwendung einer Weiterbildung dieses Verfahrens ist es, sicherzustellen, dass ein bestimmtes Disposable D (z.B. Dialysator), welches für die mehrmalige Benutzung geeignet und vorgesehen ist, nur von derselben Person P verwendet wird. Mit den oben gezeigten Verfahren kann man ein Verknüpfungsereignis zweier Verknüpfungspartner erzeugen und die Wiederholung eines identischen Ereignisses dokumentieren sowie ermitteln.

Wenn aber ein Disposable D zunächst mit einem ersten Patienten P_1 verknüpft wird, gibt es in den obigen Verfahren keinen Hinweis, dass es nicht (auf autorisierte Weise) mit einem zweiten Patienten P_2 verknüpft werden sollte.

Bei einer Variante, um nun sicherzustellen, dass ein einmal mit einem ersten Patienten P_1 verknüpfter Partner D nicht mit einem weiteren Partner P_2 verknüpft werden kann, sondern immer nur mit dem Partner P_1 (wiederholt) verknüpft werden kann, wird das Verfahren wie folgt erweitert:

Bei der allerersten Verknüpfung eines Disposable D werden zwei Werte W(n=0) und W(n=1) erzeugt und in einer gemeinsamen Datenstruktur im Distributed Ledger DL gespeichert. Dabei wird W(n=0) nur in Abhängigkeit von ID-n und ID-D erzeugt:

$$W(n=0) = f(ID\text{-}D) \; \varphi \; h \; (ID\text{-}n(n=0))$$

Aber zur Erzeugung von W(n=1) wird wieder ID-P herangezogen:

$$W(n=1) = f(ID\text{-}D) \; \varphi \; g(ID\text{-}P) \; \varphi \; h \; (ID\text{-}n(n=1))$$

Die folgenden Werte W (n>1) werden wie gewohnt erzeugt.

Wenn nun eine beliebige Instanz im Netzwerk eine ID-D scannt und das Verfahren anwendet, wird zuerst der Wert W(0) erzeugt und im distributed ledger DL danach gesucht. Wenn der Wert gefunden wird, ist klar, dass direkt dahinter die einzige zulässige Kombination von ID-D und ID-P in Form von W(1) abgelegt ist. Wenn nun ein Verknüpfungspartner P eine Verknüpfungsereignis mit genau diesem Disposable mit ID-D herbeiführen möchte, scannt das Gerät seine ID-P und errechnet den Wert W(1) zu dieser Kombination von Verknüpfungspartnern. Passt dieser Wert W(1) zu dem Wert, der hinter dem aufgefundenen Wert W(0) zu dem Disposable mit dem Identifikator ID-D gespeichert ist, so ist klar, dass es sich um denselben Verknüpfungspartner P handelt, wie bei dem dokumentierten Verknüpfungsereignis W(1).

Wenn der errechnete Wert W(1) nicht zu dem gespeicherten Wert W(1) passt, sind die beteiligten Verknüpfungspartner P nicht identisch.

Im obigen Beispiel würde das Gerät also eine Fehlermeldung ausgeben und das Gerät sperren bzw. den Start verweigern. Zusätzlich können noch weitere Maßnahmen ausgelöst werden, wie z.B. ein manuelles Überschreiben bzw. ein "manual override" zum Start erfordern (der wiederum fälschungssicher dokumentiert werden würde), eine Fehlermeldung an einen Backbone schicken, die Schwester rufen und/oder dergleichen.

In einer Weiterbildung wird die Verknüpfungslogik umgekehrt und es sind nur solche Verknüpfungen erlaubt, die es noch nicht gegeben hat.

Maximale Wiederholungsanzahl und Abbruch:

Eine Weiterbildung des Verfahrens ermöglicht, eine bestimmte Anzahl von Wiederholungen n_max zu erlauben und danach (n>n_max) keine weiteren Wiederholungen der Verknüpfungsereignisse mehr zuzulassen.

Bei einer einfachen Variante, das zu realisieren, würden die beteiligten Instanzen eine Maximalanzahl kennen - entweder lokal oder im Gerätenetzwerk oder bei einer Zentralinstanz wäre n_max abgelegt. Dieses Szenario könnte - vor allem bei lokaler Speicherung ein Angriffsrisiko bergen. In jedem Fall würden aber auch Wiederholungen über dem Maximum im Distributed Ledger DL dokumentiert und es könnte die Haftung ausgeschlossen werden.

In einer Weiterbildung ist die Anzahl n_max eine spezifische Eigenschaft des Verknüpfungspartners D und kryptologisch in dessen Identifikator ID-D enthalten, z.B. als Prüfsumme.

In einer Weiterbildung ist die Anzahl der Wiederholungen n_max spezifisch für jeden Verknüpfungspartner P festgelegt und in dessen Identifikator ID-P kodiert.

In einer Weiterbildung ist die Anzahl der Wiederholungen sowohl von P als auch von D abhängig und aus einem Raum möglicher Maxima wird in Abhängigkeit von ID-D und ID-P ein Wert bestimmt.

In einer Weiterbildung sind die Identifikatoren ID-n der Wiederholungszahl n nur für eine bestimmte Menge n bis hoch zu einem ausgewählten n_,max definiert, z.B. für n Element aus (1-10) bis n_max=10 oder für n Element aus (0-3) mit n_max=3. Versucht nun eine Instanz, einen Wert W(n>n_max) zu erzeugen, wird sie scheitern, da sie keinen Identifikator ID-n für n>n_max erzeugen kann. Es wird ein Fehler auftreten, der als "Anzahl der maximalen Wiederholungen erreicht" auf einer Benutzeroberfläche ausgegeben und/oder dokumentiert werden kann, optional gefolgt von einem Abbruch oder einer Warnung, dass die Garantie erlischt etc.

In einer Weiterbildung wird ID-n mit einer Funktion auf n gebildet, die kein Ergebnis oder kein sinnvolles Ergebnis liefert für Werte von n>n_max. Beispielsweise könnte ID-n eine Funktion t(n) aufweisen, die für n=0 bis n=n_max=10 reale Ergebnisse liefert und für natürliche Zahlen n größer 10 imaginäre Ergebnisse liefert:

$$t(n)=\sqrt{(9{,}99-n)}$$

Alternativ könnte in der Variante zur oben geschilderten Überprüfung, ob ein Verknüpfungspartner bereits verknüpft ist, unmittelbar nach der Erzeugung des Werts W (0) eine Verknüpfung mit ID-n (n= n_max) erfolgen und bei jeder weiteren Verwendung heruntergezählt werden. Bei Erreichen von n=1 wäre dann die maximal zulässige Anzahl Wiederverwendungen erreicht und beim nächsten Dekrementieren (n=0) könnte die Funktion ein ungültiges Ergebnis auswerfen.

Weitere Anwendungsbeispiele:

Der erster Anwendungsfall ist die mehrfache Verwendung z.B. eines Dialysators, der derzeit typischerweise nur für den einmaligen Gebrauch vorgesehen ist. Aus verschiedenen hygienischen und gesundheitlichen Gründen ist erwünscht, dass ein Dialysator - wenn überhaupt - nur von ein und demselben Patienten mehrfach verwendet wird. Daher ist eine Dokumentation von Verknüpfungen aus Patienten-ID und Dialysator-ID / Disposable (Einmalprodukt) - ID wünschenswert. Nicht besonders relevant ist dabei, dass dieselbe Dialysemaschine dazu verwendet wird. Es wäre aber denkbar und könnte in Weiterbildungen auch dokumentiert werden. Weitere Parameter, die dokumentiert werden könnten, sind Behandlungsdauer und weitere Behandlungsparameter, sowie eine zulässige Verwendungszeit nach der Erstverwendung. Aber das liegt nicht im Kern dieses Verfahrens. Mit dem Verfahren könnte aber auch sichergestellt werden, dass nur die ärztlich festgelegte Anzahl wiederholter Verwendungen desselben Dialysators möglich ist, z.B. fünf Wiederholungen.

Ein weiterer Anwendungsfall für dieses Verfahren ist die therapeutische Apherese. Dies ist eine gerätebasierte Behandlung zur Entfernung pathogener Substanzen aus dem Blut mit Hilfe von Filtern und Adsorbern. Der weitere im Folgenden geschilderte Anwendungsfall betrifft insbesondere die Verwendung eines Adsorbers im Rahmen des Betriebs eines Plasmafiltrationsgerätes und eines Immunapheresegerätes und dessen sichere Dokumentation.

Ein Adsorber kann als Disposable bei demselben Patienten - sogar mehrfach während einer einzelnen Behandlung - wiederverwendet werden. Jeder Patient kann zumindest einen auffrischbaren Adsorber verwenden. Als Gebrauchsgegenstand können beispielsweise zwei Adsorber im Wechsel betrieben werden. Dabei wird der erste Adsorber verwendet und der zweite zeitgleich (mit Natriumchlorid) regeneriert. Da es sich bei einem Adsorber um ein kostenintensives Produkt handelt, ist der Anreiz, das Disposable mehrfach zu verwenden hoch und zwar für eine:

- Mehrfachverwendung während der individuellen Behandlungssitzung und eine
- Mehrfachverwendung in mehreren Behandlungssitzungen.

Bei der Verwendung des Adsorbers ist es wesentlich, zu prüfen, wie lange er verwendet worden ist bzw. noch auf autorisierte Weise verwendet werden kann.

Mit dem hier vorgestellten Verfahren und System ist es grundsätzlich möglich, eine Betriebszeit des Disposables (ob Membran oder Dialysator oder ein anderer Typ von medizinischem Disposable) zu überprüfen. Verknüpfungsereignisse können zeitlich getaktet erfasst werden (z.B. pro Stunde oder pro Minute ein Verknüpfungsereignis), um die Wiederverwendung von den Verbrauchsgegenständen in den Geräten dokumentieren und/oder kontrollieren zu können. Dies hat den Hintergrund, dass eine solche Membran nicht pro Behandlung eingelegt wird, sondern für eine bestimmte Zeitdauer, die in der Regel länger als ein Behandlungszyklus ist. Daher wird ein neues Verknüpfungsereignis pro vordefinierte Zeiteinheit erfasst bzw. validiert, z.B. ein Verknüpfungsereignis pro Stunde. Das Erfassen von Verknüpfungsereignissen umfasst den Verfahrensschritt des Validierens.

Ein weiterer Anwendungsfall ist ein dialysatorähnlicher Filter, der in Behandlungsgeräten zusätzlich zur Filterung von Dialysewasser oder Dialyseflüssigkeit eingesetzt wird. Mit solchen Filtern werden Bakterien, Viren und Endotoxine aus den Flüssigkeiten herausgefiltert bzw. zurückgehalten. Diese Filter dürfen nur begrenzt eingesetzt werden. Die Begrenzung kann sich auf die Anzahl von zulässigen Wiederholungen, auf eine Zeitdauer, auf eine Begrenzung hinsichtlich der Verwendung für einen Patienten oder auf andere Aspekte beziehen. Dieser Anwendungsfall ist ein Beispiel dafür, dass kumulativ mehrere (hier: drei) unterschiedliche (Verwendungs-) Kriterien anzuwenden sind, um den Gerätebetrieb mit dem Disposable (hier: Filter) zu autorisieren. Die drei verschiedenen Kriterien für eine maximale Verwendung sind

- Maximale Anzahl. Z.B. darf ein solcher Filter maximal eine festgelegte Anzahl von chemischen Desinfektionszyklen, z.B. mittels Chlor oder Hypochlorid, erlebt haben.

- Maximale Betriebsdauer. Der Filter darf nur eine festgelegte maximale Betriebsdauer im Einsatz gewesen sein.
- Maximale Standzeit. Der Filter darf nur eine bestimmte maximale Standzeit erreichen, d.h. eine zeitliche Obergrenze ist erreicht, wenn der Einbau in ein Gerät eine bestimmte Dauer erreicht - unabhängig davon, ob er in Betrieb war oder chemisch desinfiziert wurde.

In einer bevorzugten Ausführungsform der Erfindung ist es vorgesehen, dass in einer vorbereitenden Konfigurationsphase des Verfahrens, Kriterien konfigurierbar sind, die vom Betreiber vorgegeben oder aus einem vordefinierten Menü ausgewählt sein können, und die automatisch während des Gerätebetriebs auf Einhaltung überwacht werden.

In anderen Anwendungen können auch nur zwei oder andere Kriterien für die zulässige Verwendung des Disposables definiert und geprüft werden. Dieses Ausführungsbeispiel soll illustrieren, dass das erfindungsgemäße Verfahren auch zur Dokumentation und/oder Kontrolle von mehreren Typen von Verknüpfungsereignissen verwendet werden kann. Dazu benötigt das Gerät mehrere unterschiedliche Identifikatoren - je nach Rolle: Einen ersten Identifikator für Verknüpfungsereignisse betreffend die Betriebsdauer des Verknüpfungspartners, einen zweiten Identifikator für Verknüpfungsereignisse betreffend Desinfektionsvorgänge des Verknüpfungspartners, und einen dritten Identifikator für Verknüpfungsereignisse betreffend die Standzeit des Verknüpfungspartners. Damit ist die Dokumentation aller drei Verwendungskriterien bzw. Vorgangsarten möglich. Bei dem Verfahren, mit dem eine Verwendung über die zulässige Obergrenze von Wiederholungen verhindert werden soll, ist sichergestellt, dass für alle drei Parameter überprüft wird, ob die maximale Zahl der Wiederholungen erreicht ist.

Weiterhin erstellt das Gerät bei jedem Desinfektionsvorgang ein Verknüpfungsereignis mit Maschinen-Desinfektions-Identifikator und dem Filter.

Zusätzlich erstellt das Gerät pro angefangener Zeiteinheit im Betrieb ein Verknüpfungsereignis mit Maschinen-Betriebs-Identifikator und dem Filter.

So wird also für das Pairing aus Maschinen-Identifikator-Standzeit und Filter-Identifikator eine Verknüpfung beim Einlegen in das Gerät erfasst und, wenn der Filter darin verbleibt, in einem festen zeitlichen Abstand, z.B. eine Verknüpfung pro Woche erfasst. Alternativ wird nur beim allerersten Einlegen eines Filters ein Verknüpfungsereignis mit dem Standzeit-Identifikator des Gerätes erfasst - und über einen Zeitstempel (timestamp) im Distributed Ledger ermittelt, ob der Filter noch verwendet werden darf oder nicht. Bei dem Produkt DiaSafe beträgt die maximal erlaubte Standzeit z.B. etwa 12 Wochen - ansonsten ist die Gefährdung durch Verkeimung und mikrobiologisches Wachstum zu groß. Letzterer Fall würde sicherstellen, dass bei Ausfall einer Maschine, die dann also keine weiteren Standzeit-Verknüpfungen mehr dokumentieren kann, dennoch die Standzeit nicht überschritten wird.

Obwohl der Filter in verschiedenen Geräten eingesetzt werden kann, können alle relevanten Verknüpfungsereignisse überwacht und dokumentiert werden, da alle Arten von Ereignissen mit dem eindeutigen Filter-Identifikator verknüpft werden, sodass eine Überschreitung der zulässigen Betriebsparameter dokumentiert, überprüft und ggf. verhindert werden kann.

Bei dieser Anwendung gibt es eine Verknüpfung mehrerer Ketten in der Form, dass es zu einem Filter-Identifikator mehrere Teilketten gibt, sozusagen eine pro Identifikator-Typ (Betriebszeit, Desinfektionen, Standzeit). Eine solche zusammenhängende Überprüfung ist nur möglich, wenn die Geräte, die das erfindungsgemäße Verfahren ausführen, Kenntnis darüber haben, welche Ketten zusammengehören, also in diesem Fall welche drei Ketten zu demselben Disposable gehören. In diesem Sinn müssen die Ketten also zumindest insofern miteinander verknüpft sein, dass das Gerät die relevanten Daten erfassen kann. Die Verknüpfung kann z.B. über einen Satz von Funktionen f1, f2, f3 realisiert sein, mit denen aus einer Disposable-ID ID-D drei verschiedene Header erzeugt werden - jeweils einer für Standzeit f1(ID-D), Betriebszeit f2(ID-D) und Anzahl Desinfektionen f3(ID-D). Wenn der Zusammenhang zwischen f1, f2 und f3 nachvollziehbar und systematisch ist, sind auf diese Weise auch die Ketten als verknüpft erkennbar, wenn man den Zusammenhang zwischen f1, f2 und f3 kennt. In realistischen Anwendungsfällen sollten die Funktionen in einem solchen systematischen Zusammenhang stehen.

Ein weiterer Anwendungsfall für dieses Verfahren ist die Dokumentation der Verwendung einer Membran für die Wasserreinigung mittels Umkehrosmose (R.O. für Reverse Osmosis). Gerade portable Geräte könnten eventuell Membrane verwenden, welche nur für z.B. 500 oder 5000 Betriebsstunden einsetzbar sind. In diesem Fall könnte man kleinere Einheiten dokumentieren, d.h. eine Wiederholung wäre dann nicht eine Verwendung, sondern es wird ein Eintrag zu einem aufsteigenden n pro angefangene Betriebsstunde oder pro angefangener Betriebsminute, für Durchflussmengen, etc. erstellt.

Der Vollständigkeit halber sei gesagt, dass ein simpler, aber denkbarer Fall

$W = f(\text{ID-D}) \; \varphi \; g(\text{ID-P}) \; \varphi \; k$ mit $f, g, \varphi = 1$ ist, d.h.

$$W = \text{ID-D} * \text{ID-P} * k$$

Im Folgenden wird die Erfindung anhand bestimmter Ausführungsbeispiele in Zusammenhang mit den Figuren noch genauer erläutert.

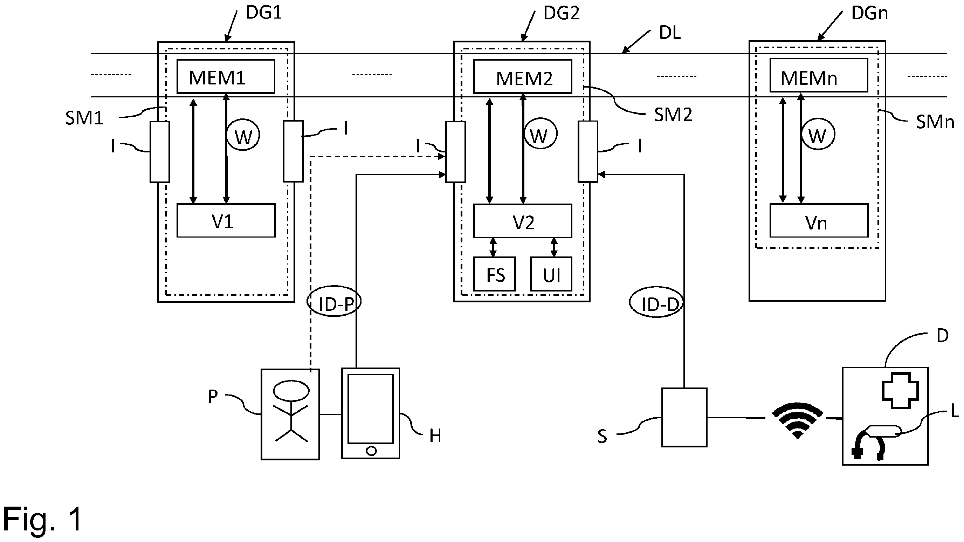

So zeigt **Fig. 1** ein Beispiel für ein Verfahren zur Absicherung von Dialysegeräten DG vor einer nicht-autorisierten Anwendung. Nicht-autorisiert ist eine Anwendung dann, wenn ein und dasselbe Disposable z.B. für unterschiedliche Patienten verwendet werden soll oder, wenn ein Disposable über die Anzahl der zulässigen Maximalanwendungen hinaus angewendet werden soll. Dabei ist eine Gruppe von Dialysegeräten DG1, DG2, ...DGn in einem Verbund aus Geräten über ein Peer-to-Peer Netzwerk verbunden. Die Dialysegeräte DG können z.B. in einem Dialysezentrum angeordnet sein. Sie können sich dabei auch weltweit verteilt in unterschiedlichen Institutionen befinden. Als Teilnehmer an den Absicherungsverfahren umfasst jedes Dialysegerät DG ein Absicherungsmodul SM. Das Absicherungsmodul SM kann direkt auf dem Dialysegerät DG oder auf einem diesem zugeordneten und mit diesem in Datenaustausch stehenden Gateway implementiert sein. Das Absicherungsmodul SM kann mit einem Freigabe-/Sperrsignalgeber interagieren, um eine Freigabe oder eine Sperrung des Dialysegerätes DG für die intendierte Behandlung mit dem Patienten P und dem Disposable D zu bewirken. Zudem kann das Absicherungsmodul SM mit einer Benutzerschnittstelle zur Darstellung des Ergebnisses oder Teilergebnisses (z.B. des berechneten Dokumentationswertes) ausgebildet sein. Das Absicherungsmodul SM kann folgende Bauteile umfassen:

1. Eine Einleseschnittstelle I. Es kann auch zwei Instanzen der Einleseschnittstelle geben, wie in Fig. 1 schematisch dargestellt, eine erste Instanz der Einleseschnittstelle I, die zum Einlesen des Patienten-Identifikators ID-P bestimmt ist und eine zweite Instanz der Einleseschnittstelle I, die zum Einlesen des Disposable-Identifikators ID-D bestimmt ist.

2. Eine digitale Verarbeitungseinheit V, die als ein Prozessor, ein Chip oder eine Schaltkreisanordnung ausgebildet sein kann und dazu dient, auf Basis der eingelesenen Identifikatoren ID-P, ID-D (bzw. ID-M, ID-RO) das Sicherungsverfahren auszuführen und die mit einem lokalen Speicher MEM des Gerätes DG und mit der verteilten Speicherstruktur DL in Datenaustausch steht, sowie ggf. mit weiteren Modulen.

3. Den Speicher MEM;

4. Optional können auf dem Dialysegerät neben den medizinischen Betriebsmodulen noch weitere Prozessoren und weitere Module bereitgestellt werden. Deshalb ist die Eingrenzungslinie der vorstehend genannten Module in Fig. 1 als Strichpunktlinie dargestellt.

Alle Speicher MEM1, MEM2, MEMn der jeweiligen Dialysegeräte DG1, DG2, ...DGm bilden die verteilte Speicherstruktur, die als Distributed Ledger ausgebildet sein kann.

Der Patient P muss sich zunächst am Absicherungsmodul SM bzw. am Dialysegerät DG anmelden und identifizieren und dem Dialysegerät DG seinen (ersten) Identifikator ID-P bereitstellen. Dazu stehen ihm grundsätzlich unterschiedliche Optionen zur Verfügung. So kann er z.B. eine App auf seinem mobilen Gerät H (Smartphone, Tablet) aktivieren bzw. aufrufen, die ihn zur Eingabe seiner Patienten-Identifikators ID-P auffordert. Andernfalls kann das Gerät H und/oder das Dialysegerät DG mit einem Lesemittel oder einer Vorrichtung (Codescanner) ausgebildet sein, um den Patienten-Identifikator ID-P als Code z.B. von einer Patienten- oder Gesundheits-Karte auszulesen. Der auf der Karte befindliche Code (z.B. Barcode) entspricht dann einer physikalischen "Kennzeichnung" des Patienten P, die zur weiteren Verarbeitung dann in den digitalen den Patienten identifizierenden Identifikator ID-P transformiert wird und diesem entspricht. Weiterhin kann er seinen ersten Identifikator ID-P auch direkt über eine Benutzerschnittstelle UI sozusagen manuell an dem Gerät DG eingeben. Dies ist in Fig.1 mit der gestrichelten Linie dargestellt.

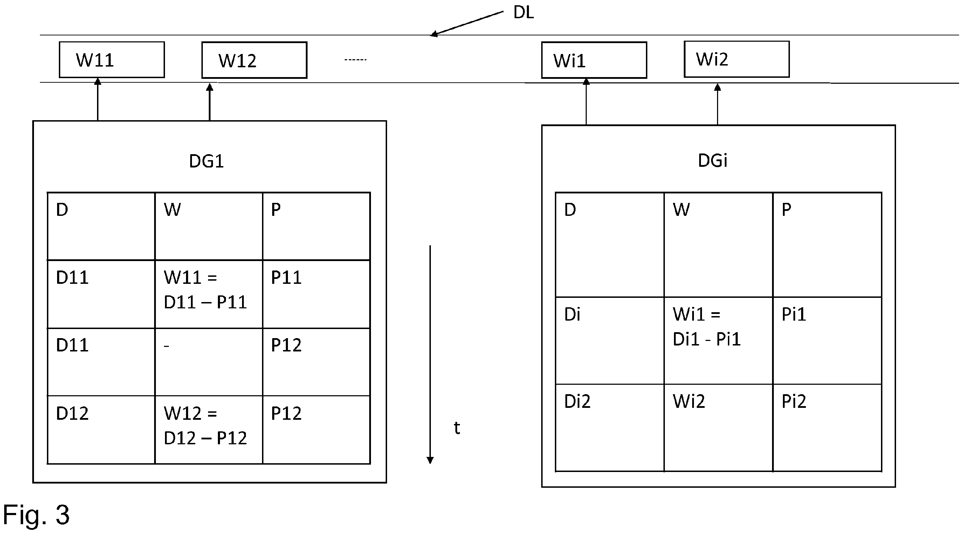

Auf entsprechende Weise wird der zweite Identifikator ID-D des Disposables D erfasst. Dazu kann das Disposable D mit einer RFID-Kennzeichnung oder einem andersartig gestalteten Label - in Fig. 1 mit dem Bezugszeichen L gekennzeichnet - ausgebildet sein, das von einem Lesegerät, hier Scanner S, erfasst und in Form des digitalen zweiten Identifikators ID-D über die Einleseschnittstelle I der Verarbeitungseinheit V zur weiteren Verarbeitung zugeführt wird. Nachdem der erste und zweite Identifikator ID-P, ID-D über die Einleseschnittstelle I eingelesen worden sind, werden sie der Verarbeitungseinheit V bereitgestellt, die eine erste Rechenvorschrift RV1 auf den ersten Identifikator ID-P und eine zweite Rechenvorschrift RV2 auf den zweiten Identifikator ID-D anwendet, um die so entstandenen beiden Teilergebnisse mit einer dritten Rechenvorschrift (in Fig. 3 als RV3 oder im Text auch mit $\varphi$ bezeichnet) zu verarbeiten, um den Dokumentationswert W zu berechnen.

Wie in Fig. 1 ersichtlich, kann das Dialysegerät DG und insbesondere das Absicherungsmodul SM (in Fig. 1 beispielhaft dargestellt) noch eine Benutzerschnittstelle UI umfassen und eine Freigabe- und/oder Sperreinheit FS, die das Gerät DG nur bei autorisierter Verknüpfung für den Betrieb mit den jeweiligen Verknüpfungspartnern D, P freischaltet. Falls keine Autorisierung stattfinden kann, kann der Gerätebetrieb zumindest für die beiden jeweiligen Verknüpfungspartner D, P gesperrt werden. Dazu kann ein entsprechender Hinweis auf der Benutzeroberfläche UI ausgegeben werden, der den Grund für die Sperrung kennzeichnet (z.B. "Gebrauchsgegenstand wurde bereits für einen anderen Patienten verwendet" oder "Zulässige Höchstzahl von Verwendungswiederholungen für den Gebrauchsgegenstand ist erreicht").

Soll das Verfahren als reines Dokumentationsverfahren angewendet werden, so kann der so berechnete Dokumentationswert W nun direkt in den Speicher MEM geschrieben werden.

Soll das Verfahren als Absicherungs- und Autorisierungsverfahren verwendet werden, so wird der berechnete Dokumentationswert W nicht direkt in den Speicher MEM geschrieben werden, sondern es erfolgt erst eine Validierung. Die Validierung kann mittels eines Konsensus-Algorithmus ausgeführt werden, der einen Zugriff auf die verteilte Speicherstruktur DL umfasst, um zu prüfen, ob der Dokumentationswert W in Bezug auf den Gebrauchsgegenstand bereits in der Speicherstruktur DL abgelegt ist und wenn ja, mit welchem Wiederholungswert. Je nachdem, welche Regeln in einer Konfigurationsphase im Vorfeld konfiguriert worden sind, insbesondere wenn er noch nicht gespeichert ist (neues Verknüpfungsereignis) oder wenn er für denselben Patienten aber innerhalb der maximal zulässigen Wiederholungsrate gespeichert worden ist (zulässige Wiederverwendung) gilt der jeweilige Dokumentationswert W als validiert; andernfalls nicht. Nur bei erfolgreicher Validierung erfolgt ein synchronisiertes Speichern des Dokumentationswert W in die Distributed Ledger Struktur DL und der Gerätebetrieb kann für die jeweiligen Verknüpfungspartner D, P autorisiert werden.

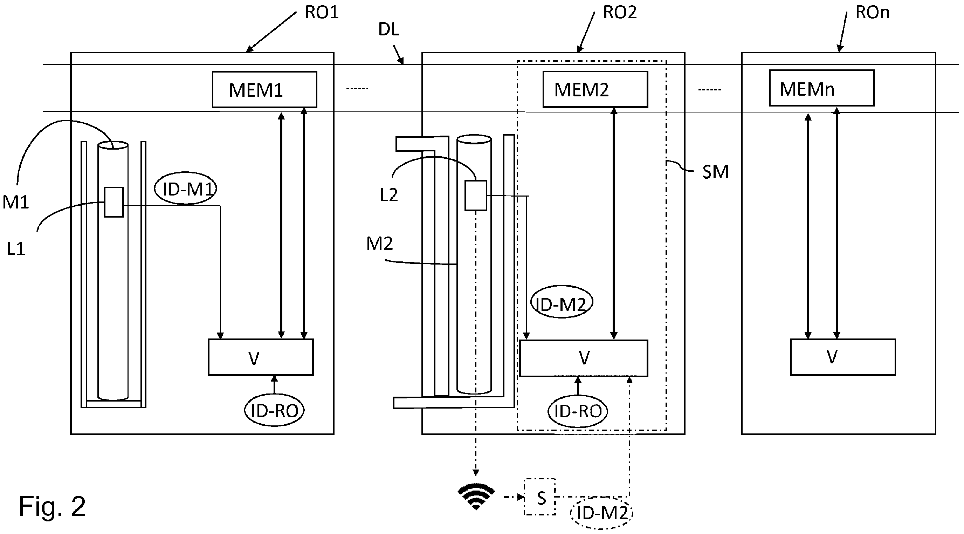

**Fig. 2** zeigt ein weiteres Ausführungsbeispiel. Hier soll überwacht werden, ob eine Umkehrosmoseanlage RO auf zulässige Weise betrieben wird. Die Umkehrosmoseanlage RO dient zur Bereitstellung von aufbereitetem, gereinigtem Wasser zur Verwendung für ein Dialysegerät DG. Sie basiert auf dem Prinzip der Umkehrosmose und umfasst dazu Pumpen und Filter, Behältnissen für die Flüssigkeiten und eine Membran M als Gebrauchsgegenstand. Die Bestandteile der Anlage RO sind in Fig: 2 nur schematisch dargestellt. Mit dem Absicherungsverfahren wird überprüft, ob die dabei verwendeten und in dem Gerät RO verwendeten Verbrauchsgegenstände, wie die Membran M, noch verwendet werden dürfen und noch nicht zu oft verwendet worden sind. Dazu ist die Umkehrosmoseanlage RO mit einer Verarbeitungseinheit V ausgebildet und der Gebrauchsgegenstand, z.B. die Membran M, ist mit einem physikalischem Label L gekennzeichnet (Barcode, Strichcode, RFID-Code etc.). Dem Label L ist auf eineindeutige Weise der erste Identifikator ID-M, ID-M1, ID-M2 etc. zugeordnet bzw. kann n diesen transformiert werden. Alternativ und in Fig. 2 mit einer Strich-Punkt-Linie dargestellt, kann das Label L von einem Scanner S ausgelesen und in Form des ersten Identifikators ID-M2 der Verarbeitungseinheit V zur weiteren Verarbeitung zugeführt werden. Der erste Identifikator ID-M ist ein digitaler Datensatz und wird von der Verarbeitungseinheit V mittels bereitgestellter Algorithmen verarbeitet. Des Weiteren wird der Verarbeitungseinheit V der zweite Identifikator ID-RO der RO-Anlage zugeführt, um die Rechenvorschriften RV1, RV2, RV3 auf die Datensätze anzuwenden.

Das Label L der Membran M wird erfasst (z.B. über einen Scanner S) und wird als erster Identifikator ID-M eingelesen und als zweiter Identifikator wird der ID-Code ID-RO der Umkehrosmoseanlage RO eingelesen. Nun wird im Wesentlichen dasselbe Verfahren, wie vorstehend in Zusammenhang mit Fig. 1 beschrieben, ausgeführt, um den Gerätebetrieb der Umkehrosmoseanlage RO mit der Membran M zu autorisieren oder zu sperren.

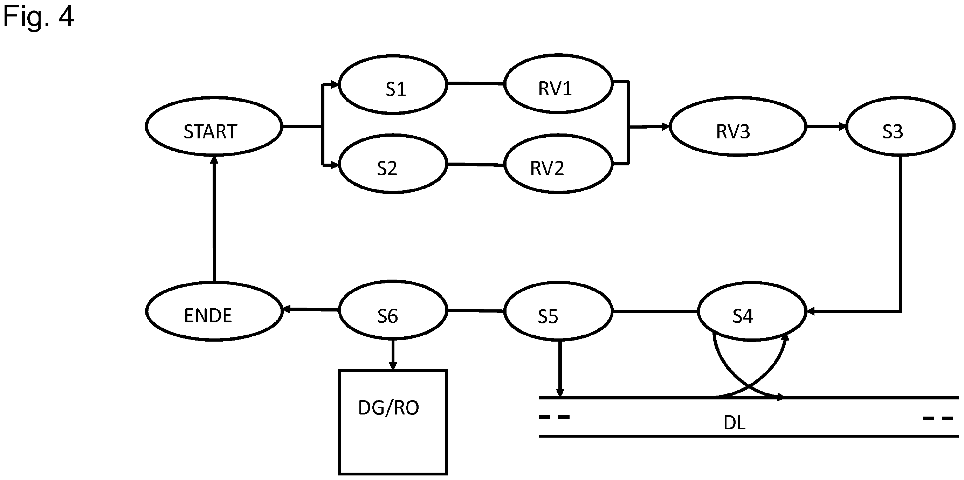

In den oben genannten Beispielen ist es für den Fachmann selbstverständlich, dass der erste und zweite Identifikator auch ausgetauscht sein können. Wie auch in Fig. 4 angedeutet, können das Erfassen der beiden Identifikatoren ID-P, ID-D, ID-RO, ID-M auch parallel oder in einer anderen zeitlichen Reihenfolge ausgeführt werden.

Wie in den Figuren 1 und 2 beispielhaft für die ersten Geräte DG, RO angedeutet, wird der berechnete Dokumentationswert W zunächst in einem lokalen Pufferspeicher zwischengespeichert. Dann kann optional eine Validierung ausgeführt werden, um der Gerätebetrieb mit den jeweiligen Verknüpfungspartnern D, M autorisiert ist. Dies kann durch einen Zugriff auf die verteilte Datenstruktur der distributed ledger DL ausgeführt werden. Damit kann erreicht werden, dass nicht zulässige Verknüpfungsversuche gar nicht erst in der verteilten Datenstruktur DL gespeichert werden, sondern nur solche die erfolgreich validiert werden konnten.

**Fig. 3** zeigt in einer schematischen Darstellung den Datenaustausch und die Zuordnungen der Datensätze. Auf einem ersten Gerät DG1 werden nacheinander unterschiedliche Patienten P mit unterschiedlichen Disposablesätzen D behandelt. Die Zeit ist in Fig. 3 von oben nach unten entsprechend der Pfeilrichtung aufgetragen. So verwendet ein erster Patient P11 an dem ersten Gerät DG1 ein Disposable D11. Der Dokumentationswert W11 wird wie oben beschrieben berechnet und durch den Konsensus-Algorithmus validiert. Da er noch nicht gespeichert ist, wird er in die DL-Struktur DL eingetragen. Danach möchte ein anderer Patient P12 (in diesem Beispiel an demselben Dialysegerät DG1) dasselbe Disposable D11 verwenden. Der berechnete Dokumentationswert W wird nicht gespeichert, da er nicht validiert werden konnte, da dasselbe Disposable bereits für einen anderen Patienten (P11) verwendet worden ist. Nun möchte ein weiterer Patient P13 ein Disposable D12 verwenden. Dies wird als zulässig bewertet und der Wert 12 wird in die Distributed Ledger DL gespeichert und so weiter.

Alle Geräte DG stehen in Datenaustausch und greifen auf die verteilte Distributed Ledger Struktur DL zu. Ein i-tes Dialysegerät DGi wird von einem Patienten Pi1 mit einem Disposable Di1 angewendet. Dies ist als zulässig berechnet worden, so dass der Wert Wi1 in die Distributed Ledger DL gespeichert wird. Ein zweiter Patient Pi2 möchte das Disposable Di2 verwenden und dementsprechend wird die Distributed Ledger DL nach dem berechneten Wert Wi2 durchsucht.

Für den Fall i=1 würde die Validierung in dem obigen Beispiel fehlschlagen, da das Disposable D11 bereits an dem ersten Gerät DG1 von dem ersten Patienten P11 verwendet worden ist. Dies wird unmittelbar an dem Gerät DGi, das sich z.B. in Deutschland befinden kann, erfasst, obwohl das erste Gerät DG1 z.B. in Japan befindlich ist.

Für den Fall i=2 würde die Autorisierung erfolgreich sein, da die jeweiligen Verknüpfungsereignisse noch nicht in der Distributed Ledger DL gespeichert sind und somit eine neue Verknüpfung vorliegt.

Im Unterschied zur Validierung kann die Autorisierung noch weitere Überprüfungen umfassen (z.B. unter Zugriff auf eine Regelbasis noch weitere Kriterien, die in Form von Regeln abgelegt sind, zu überprüfen, wie z.B. die Art der intendierten Behandlung für die jeweiligen Verknüpfungspartner etc.).

**Fig. 4** zeigt den typischen Ablauf des Verfahrens eines Ausführungsbeispiels in einem Ablaufdiagramm. Nach dem Start des Verfahrens wird in Schritt S1 der erste Identifikator ID-P eingelesen. In Schritt S2 wird der zweite Identifikator ID-D eingelesen. Schritt S1 und S2 können auch in einer umgekehrten Reihenfolge ausgeführt werden. Die erste Rechenvorschrift RV1 wird auf den ersten Identifikator ID-P angewendet und die zweite Rechenvorschrift RV2 wird auf den zweiten Identifikator ID-D angewendet, um Teilergebnisse bereitzustellen, die mittels einer dritten Rechenvorschrift RV3 verarbeitet werden, um in Schritt S3 den Dokumentationswert W bereitzustellen bzw. zu berechnen. In Schritt S4 kann das Validieren des berechneten Dokumentationswertes W durch Ausführen des Konsensus-Algorithmus unter Zugriff auf die Distributed Ledger DL erfolgen. Bei erfolgreicher Validierung erfolgt in Schritt S5 das synchronisierte Speichern des Wertes W in die Distributed Ledger DL und in Schritt S6 kann das Gerät DG mit dem jeweiligen Disposable D als Verknüpfungspartner freigeschaltet werden. Dies erfolgt durch gezielte Ansteuerung des Gerätes DG, RO. Danach kann das Verfahren iterativ ausgeführt oder beendet werden. Der vorstehend geschilderte Ablauf kann sowohl für die Absicherung eines Dialysegerätes DG mit einer Verwendung von Disposables D als auch für die Absicherung einer Umkehrosmoseanlage RO mit einer Verwendung von Membranen M angewendet werden.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Für einen Fachmann ist es insbesondere offensichtlich, dass die Erfindung nicht nur für Dialysegeräte DG und Umkehrosmoseanlagen RO angewendet werden kann, sondern auch für andere medizintechnische Geräte, die Verbrauchsgegenstände verwenden, deren Verwendung an oder mit dem jeweiligen Gerät überprüft werden soll. Des Weiteren können die Bauteile des Absicherungsmoduls SM auch auf mehrere physikalischen Produkte verteilt realisiert werden kann.

Der Schutzbereich der vorliegenden Erfindung ist durch die folgenden Ansprüche gegeben und wird durch die in der Beschreibung erläuterten oder den Figuren gezeigten Merkmale nicht beschränkt.

**Patentansprüche**

1. Verfahren zur Absicherung eines Gerätes (DG, RO) im medizinischen Umfeld vor einem nicht autorisierten Betrieb des Gerätes (DG, RO), wobei das Gerät (DG, RO) (DG, RO) über ein Netzwerk mit anderen Geräten (DG, RO) verbunden ist und wobei die Autorisierung abhängig ist von einem Verknüpfungsereignis zwischen zumindest zwei Verknüpfungspartnern im Rahmen eines Gerätebetriebs, wobei einer der Verknüpfungspartner ein Gebrauchsgegenstand (D, M) ist, der bei dem Gerätebetrieb verwendet wird, mit folgenden Verfahrensschritten:

   - Einlesen (S1) eines ersten Identifikators (ID-P, ID-M) zur eindeutigen Identifikation eines ersten Verknüpfungspartners (P, M) und eines zweiten Identifikators (ID-D, ID-RO) zur eindeutigen Identifikation eines zweiten Verknüpfungspartners (D, RO);
   - Anwenden (S2) zumindest einer ersten Rechenvorschrift (RV1) auf den eingelesenen ersten Identifikator (ID-P, ID-M) und Anwenden einer zweiten Rechenvorschrift (RV2) auf den zweiten Identifikator (ID-D, ID-RO) zur Berechnung (S3) eines Dokumentationswertes (W), der ein Verknüpfungsereignis zwischen dem ersten und zweiten Verknüpfungspartner eindeutig repräsentiert;
   - Veranlassen einer synchronisierten Speicherung (S5) des berechneten Dokumentationswertes (W) in Speichern von Geräten des Netzwerkes und
   - Absichern des Gerätebetriebs für den ersten und zweiten Verknüpfungspartner (P-D, M-RO).

2. Verfahren nach dem vorangehenden Verfahrensanspruch 1, bei dem das synchronisierte Speichern unter Verwendung einer verteilten Speicherstruktur erfolgt, insbesondere einer Distributed Ledger Struktur (DL).

3. Verfahren nach dem unmittelbar vorangehenden Verfahrensanspruch, bei dem die Distributed Ledger Struktur (DL) implementiert ist, indem ein Datenblock zumindest den berechneten Dokumentationswert (W) umfasst und keinen Verweis auf andere Blöcke enthält.

4. Verfahren nach einem der vorangehenden Verfahrensansprüche, bei dem das Verfahren zusätzlich ein Validieren (S4) des berechneten Dokumentationswertes (W) durch Anwendung eines Konsensus-Algorithmus umfasst, so dass das Veranlassen und das Absichern nur bei erfolgreicher Validierung ausgeführt werden.

5. Verfahren nach einem der vorangehenden Verfahrensansprüche, bei dem ein Konsensus-Algorithmus zum Validieren (S4) des berechneten Dokumentationswertes (W) ausgeführt wird und/oder wobei der Konsensus-Algorithmus folgenden Verfahrensschritt umfassen kann:

   - Zugreifen auf einen Speicher (MEM) einer verteilten Speicherstruktur (DL) eines Gerätes (DG, RO), um für einen vorgegebenen Dokumentationswert (W) zu prüfen, ob dieser bereits gespeichert ist und nur verneinendenfalls und/oder bei Unterschreiten einer vordefinierbaren Anzahl von zulässigen Wiederholungswerten:
   - Validieren des Dokumentationswertes (W).

6. Verfahren nach einem der vorangehenden Verfahrensansprüche, bei dem das Gerät (DG, RO) für den intendierten Betrieb mit den beiden jeweiligen Verknüpfungspartnern bei nicht erfolgreicher Validierung (S4) und/oder fehlender Autorisierung (S6) gesperrt wird.

7. Verfahren nach einem der vorangehenden Verfahrensansprüche, bei dem ein Validieren (S4) und/oder ein Autorisieren (S6) des Verknüpfungsereignisses zeitlich getaktet und/oder ereignisbasiert ausgeführt wird.

8. Verfahren nach einem der vorangehenden Verfahrensansprüche, bei dem die Berechnung des Dokumentationswertes (W) das Berechnen eines dritten Identifikators als Wiederholungswert umfasst, der eine Anzahl an Wiederholungen für ein Verknüpfungsereignis zwischen dem ersten und zweiten Verknüpfungspartner eindeutig repräsentiert, so dass Wiederholungen von Verknüpfungsereignissen differenzierbar dokumentiert werden.

9. Verfahren nach einem der vorangehenden Verfahrensansprüche, bei dem die Berechnung des Dokumentationswertes (W) das Anwenden einer dritten Rechenvorschrift (RV3) auf ein erstes Teilergebnis, erhalten durch Anwenden der ersten Rechenvorschrift (RV1) auf den ersten Identifikator (ID-P, ID-M), und ein zweites Teilergebnis, erhalten durch Anwenden der zweiten Rechenvorschrift (RV2) auf den zweiten Identifikator (ID-D, ID-RO), zur Bereitstellung eines dritten Teilergebnisses und gegebenenfalls unter Anwendung einer weiteren Verknüpfungsfunktion auf das dritte Teilergebnis erfolgt.

10. Verfahren nach einem der vorangehenden Verfahrensansprüche, bei dem alle Geräte (DG, RO) dieselben Rechenvorschriften zur Berechnung des Dokumentationswertes (W) anwenden.

11. Computerprogramm mit Programmcode, der für das Ausführen eines Verfahrens nach einem der vorhergehenden Verfahrensansprüche geeignet ist, wenn das Computerprogramm auf einem Computer oder einer Computer-basierten Verarbeitungseinheit eines Gerätes (DG, RO) oder einem Absicherungsmodul (SM) ausgeführt wird.

12. Absicherungsmodul (SM) für ein Gerät (DG, RO) für das medizinische Umfeld, auf das eine Applikation geladen und ausgeführt werden kann, um mit folgenden Bauteilen ein Verfahren gemäß den vorangehenden Verfahrensansprüchen auszuführen, wenn die Applikation auf dem Absicherungsmodul (SM) ausgeführt wird:

   - eine Einleseschnittstelle (I) zum Einlesen eines ersten Identifikators (ID-P, ID-M) und eines zweiten Identifikators (ID-D; ID-RO);
   - eine elektronische Verarbeitungseinheit (V), die ausgebildet ist, ein Verfahren nach einem der vorangehenden Verfahrensansprüche auszuführen;
   - einer Schnittstelle zu einem Speicher (MEM) oder mit einem Speicher (MEM) zur Speicherung des berechneten Dokumentationswertes (W);
   - einer Schnittstelle zu einem Netzwerk, über das die Geräte (DG, RO) in Datenaustausch stehen.

13. Absicherungsmodul (SM) gemäß dem unmittelbar vorangehenden Anspruch, wobei das Absicherungsmodul (SM) mit einer Schnittstelle zu weiteren Absicherungsmodulen (SM) von anderen Geräten (DG, RO) des Netzwerkes ausgebildet ist.

14. Gerät (DG, RO) mit einem Absicherungsmodul (SM), gemäß einem der vorangehenden auf das Absicherungsmodul (SM) gerichteten Ansprüche.

**15.** System zur Absicherung eines Gerätes (DG, RO) im medizinischen Umfeld vor einem nicht autorisierten Betrieb des Gerätes, wobei eine Autorisierung abhängig ist von einem Verknüpfungsereignis zwischen zwei Verknüpfungspartnern im Rahmen eines Gerätebetriebs, wobei einer der Verknüpfungspartner ein Gebrauchsgegenstand ist, der bei dem Gerätebetrieb verwendet wird, wobei das Gerät (DG, RO) in einem verteilten Geräteverbund betrieben wird und wobei jeweils ein Gerät (DG, RO) ein Absicherungsmodul (SM) gemäß einem der vorangehenden auf das Absicherungsmodul (SM) gerichteten Ansprüche umfasst.

**16.** System nach dem vorangehenden Anspruch, bei dem die Speicher (MEM) der Geräte (DG, RO) eine verteilte Speicherstruktur (DL) bilden.

**17.** System nach einem der vorangehenden auf das System gerichteten Ansprüche, bei dem ein erster Verknüpfungspartner ein Patient (P) ist, dem ein ihn eineindeutig identifizierender erster Identifikator (ID-P) zugeordnet ist und bei dem ein zweiter Verknüpfungspartner ein medizinischer Gebrauchsgegenstand (D) ist, der mit einer Kennzeichnung gekennzeichnet ist, wobei der Kennzeichnung auf eineindeutige Weise ein zweiter Identifikator (ID-D) zugeordnet ist und bei dem das Verknüpfungsereignis die Verwendung des jeweiligen Gebrauchsgegenstandes (D) für den jeweiligen Patienten (P) bei einer Behandlung an einem bestimmten medizinischen Gerät (DG) repräsentiert.

**18.** System nach einem der vorangehenden Ansprüche, bei dem ein erster Verknüpfungspartner eine Membran (M) ist, wobei die Membran (M) mit einer sie eineindeutig identifizierenden Kennzeichnung ausgebildet ist, wobei der Kennzeichnung auf eineindeutige Weise ein erster Identifikator (ID-M) zugeordnet ist und bei dem ein zweiter Verknüpfungspartner eine Umkehrosmose-Anlage (RO) ist, die mit einer sie eineindeutig identifizierenden Kennzeichnung ausgebildet ist, der auf eineindeutige Weise ein zweiter Identifikator (ID-RO) zugeordnet ist und bei dem das Verknüpfungsereignis die Verwendung der Membran (M) in der Umkehrosmose-Anlage (RO) repräsentiert.

Fig. 1

Fig. 2

RO1    DL    RO2    ROn

MEM1    MEM2    MEMn

SM

ID-M1    L2

M1    M2

L1    ID-M2

V    V    V

ID-RO    ID-RO

S    ID-M2

EP 3 699 923 A1

24

DL

| W11 | | W12 | | ------ | | Wi1 | | Wi2 |

**DG1**

| D | W | P |
|------|------------------|-----|
| D11 | W11 = D11 – P11 | P11 |
| D11 | - | P12 |
| D12 | W12 = D12 – P12 | P12 |

t

**DGi**

| D | W | P |
|-----|------------------|-----|
| | | |
| Di | Wi1 = Di1 - Pi1 | Pi1 |
| Di2 | Wi2 | Pi2 |

Fig. 3

EP 3 699 923 A1

## Fig. 4

Europäisches
Patentamt

European
Patent Office

Office européen
des brevets

**EUROPÄISCHER RECHERCHENBERICHT**

Nummer der Anmeldung

EP 19 15 8317

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2018/096121 A1 (GOERINGER STEVEN JOHN [US] ET AL) 5. April 2018 (2018-04-05) * Absätze [0006], [0019], [0025], [0026], [0029], [0030], [0033], [0036], [0037], [0039], [0041], [0042], [0047], [0051], [0052]; Ansprüche 6,13; Abbildung 1 * ----- | 1-18 | INV. G16H40/40 G16H40/60 G06F21/00 H04L29/08 |
| X | US 2017/295157 A1 (CHAVEZ DAVID [US] ET AL) 12. Oktober 2017 (2017-10-12) * Absätze [0036] - [0039], [0047], [0060] - [0062]; Abbildungen 6,7 * ----- | 1 | |
| X | ALBLOOSHI M ET AL: "Blockchain-based Ownership Management for Medical IoT (MIoT) Devices", 2018 INTERNATIONAL CONFERENCE ON INNOVATIONS IN INFORMATION TECHNOLOGY (IIT), IEEE, 18. November 2018 (2018-11-18), Seiten 151-156, XP033495442, DOI: 10.1109/INNOVATIONS.2018.8606032 [gefunden am 2019-01-08] * pp. 151 bis 152, Abschnitt "Introduction"; pp. 153 bis 155, Abschnitt "IV. Blockchain-based System for Device Ownership Management"; Abbildungen 3,4 * ----- | 1 | RECHERCHIERTE SACHGEBIETE (IPC) G16H G06F H04L |

Der vorliegende Recherchenbericht wurde für alle Patentansprüche erstellt

| Recherchenort | Abschlußdatum der Recherche | Prüfer |
|---|---|---|
| München | 6. August 2019 | Heidrich, Alexander |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

......................................................................

& : Mitglied der gleichen Patentfamilie, übereinstimmendes
Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 19 15 8317

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-08-2019

| Im Recherchenbericht angeführtes Patentdokument | Datum der Veröffentlichung | Mitglied(er) der Patentfamilie | Datum der Veröffentlichung |
|---|---|---|---|
| US 2018096121 A1 | 05-04-2018 | KEINE | |
| US 2017295157 A1 | 12-10-2017 | CN 107277810 A | 20-10-2017 |
| | | JP 2017187777 A | 12-10-2017 |
| | | US 2017295157 A1 | 12-10-2017 |

Für nähere Einzelheiten zu diesem Anhang : siehe Amtsblatt des Europäischen Patentamts, Nr.12/82

EPO FORM P0461

**EP 3 699 923 A1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2015024647 A2

### In der Beschreibung aufgeführte Nicht-Patentliteratur

- **C CACHIN ; M VUKOLIC.** *Blockchain consensus protocols in the wild,* 2017

- **A. S. TANENBAUM ; M. VAN STEEN.** Verteilte Systeme. 2007